(19) Europäisches Patentamt
European Patent Office
Office européen des brevets

(11) Publication number: **0 131 684 B1**

(12) # EUROPEAN PATENT SPECIFICATION

(45) Date of publication of patent specification: **11.03.92**

(51) Int. Cl.5: **A01N 43/64**, C09D 5/14

(21) Application number: **84103222.0**

(22) Date of filing: **18.08.81**

(60) Publication number of the earlier application in accordance with Art.76 EPC: **0 047 594**

(54) **Use of triazolylethanol derivatives and their compositions as non-agricultural fungicides.**

(30) Priority: **18.08.80 GB 8026884**

(43) Date of publication of application: **23.01.85 Bulletin 85/04**

(45) Publication of the grant of the patent: **11.03.92 Bulletin 92/11**

(84) Designated Contracting States: **AT BE CH DE FR GB IT LI LU NL SE**

(56) References cited:
**EP-A- 0 011 768**
**EP-A- 0 011 769**
**EP-A- 0 015 756**
**EP-A- 0 019 189**
**DE-A- 2 654 890**
**DE-A- 2 737 489**
**GB-A- 2 064 520**

(73) Proprietor: **IMPERIAL CHEMICAL INDUSTRIES PLC**
**Imperial Chemical House, Millbank**
**London SW1P 3JF(GB)**

(72) Inventor: **Parry, Keith Peter**
**7 Juniper Drive, Off Ray Park Road,**
**Maidenhead, Berkshire(GB)**
Inventor: **Rathmell, William George**
**"Culvers", 10 Gypsy Lane,**
**Wokingham, Berkshire(GB)**
Inventor: **Worthington, Paul Anthony**
**4 Oakhurst Road, Maidenhead Court Road,**
**Maidenhead, Berkshire(GB)**

(74) Representative: **Alner, Henry Giveen Hamilton et al**
**Imperial Chemical Industries PLC Legal Department: Patents P.O. Box 6 Bessemer Road**
**Welwyn Garden City Herts, AL7 1HD(GB)**

**EP 0 131 684 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to triazole compounds useful as fungicides for the treatment of wood, hides, leather and paint films.

Triazole derivates having plant fungicidal activity are known from DE-A-2654890; and from EP-A-0019189 (published on 26 November 1980).

Triazole derivatives having anti-mycotic activity are known from EP-A-0011768.

Triazole derivatives having utility as industrial fungicides are known from DE-A-2737489.

The present invention provides a method of treating wood, hides, leather and paint films which comprises treating such materials with a compound of general formula (I):

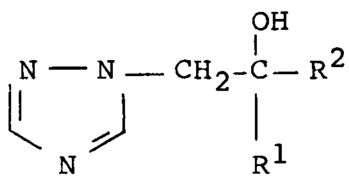

wherein $R^1$ is $C_{1-6}$ alkyl, cyclobutyl, cyclopropyl, cyclopentyl, cyclohexyl or phenyl, and $R^2$ is phenyl or benzyl; the phenyl or $R^1$ and the phenyl or phenyl moiety of the benzyl of $R^2$ being optionally substituted with halogen, $C_{1-5}$ alkyl, $C_{1-4}$ alkoxy, halo-$C_{1-4}$ alkyl, halo-$C_{1-4}$ alkoxy, nitro, phenyl, phenoxy, benzyl, optionally halo-substituted benzyloxy, alkylenedioxy, haloalkylenedioxy, amino, mono-or di-$C_{1-4}$ alkylamino, hydroxy, morpholino or carboxy or an alkyl ester thereof, and/or the alkyl moiety of the benzyl is optionally substituted with one $C_{1-2}$ alkyl; or an acid addition salt, ester or metal complex thereof.

In a further aspect the invention provides a method as defined above wherein $R^1$ is $C_{1-4}$ alkyl and $R^2$ is optionally substituted benzyl.

In another aspect the invention provides a method as described above wherein $R^1$ is optionally substituted phenyl and $R^2$ is optionally substituted benzyl.

In a yet further aspect the invention provides a method as defined above wherein each of $R^1$ and $R^2$, which may be the same or different, is optionally substituted phenyl.

In a still further aspect the invention provides a method as defined above wherein $R^1$ is $C_{1-4}$ alkyl and $R^2$ is optionally substituted phenyl, especially halophenyl and particularly chlorophenyl.

When $R^1$ is alkyl it can be a straight or branched chain group having 1 to 6, e.g. 1 to 4, carbon atoms; examples are methyl, ethyl, propyl (n- or iso-propyl), butyl (n-, sec-, iso- or t-butyl), pentyl (e.g. n-pentyl) and hexyl (e.g. n-hexyl). It is preferably butyl, especially t-butyl, and especially so when $R^2$ is chlorophenyl.

The compounds of the invention can contain chiral centres. Such compounds are generally obtained in the form of racemic mixtures. However, these and other mixtures can be separated into the individual isomers by methods known in the art.

Examples of suitable substituents for the phenyl and for the phenyl moiety of the benzyl are halogen (e.g. fluorine, chlorine or bromine), $C_{1-5}$ alkyl [e.g. methyl, ethyl, propyl (n- or iso-propyl) and butyl (n-, sec-, iso-or t-butyl], $C_{1-4}$ alkoxy (e.g. methoxy and ethoxy), halo- $C_{1-4}$ alkyl (e.g. trifluoromethyl or 1, 1, 2, 2-tetrafluoroethyl), halo- $C_{1-4}$ alkoxy (e.g. trifluoromethoxy or 1, 1, 2, 2-tetrafluoroethoxy), nitro, phenyl, phenoxy, benzyl, benzyloxy (optionally ring substituted with halogen), alkylenedioxy, haloalkylenedioxy (e.g. difluoromethylenedioxy), amino, mono- or di- $C_{1-4}$ alkylamino (e.g. dimethylamino), hydroxy, morpholino and carboxy (and alkyl esters thereof). The alkyl moiety of the benzyl can be substituted with for example one alkyl (e.g. methyl or ethyl). Suitably the phenyl and benzyl are unsubstituted or substituted with 1, 2 or 3 ring substituents as defined above. Preferably the benzyl and phenyl have a single ring substituent in the 2- or 4- position. Examples of these groups are phenyl, benzyl, $\alpha$-methylbenzyl, 2-, 3- or 4-chlorophenyl, 2,4- or 2,6-dichlorophenyl, 2-, 3- or 4-fluorophenyl, 2,4- or 2,6-difluorophenyl, 2-, 3- or 4-bromophenyl, 2-chloro-4-fluorophenyl, 2-fluoro-4-chlorophenyl, 2-chloro-6-fluorophenyl, 2-, 3- or 4-methoxyphenyl, 2, 4-dimethoxyphenyl, 2-, 3- or 4-ethoxyphenyl, 2-, 3- or 4-nitrophenyl, 2-chloro-4-nitrophenyl, 2-chloro-5-nitrophenyl, 2-, 3- or 4-methylphenyl, 2, 4-dimethylphenyl, 2-, 3- or 4-t-butylphenyl, 2-, 3- or 4-trifluoromethylphenyl, 2-, 3- or 4-trifluoromethoxyphenyl, 2-, 3- or 4-(1,1,2,2-tetrafluoroethyl)phenyl, 2, 3-(difluoro-methylenedioxy)phenyl, 2-fluoro-4-methoxyphenyl, 2-methoxy-4-fluorophenyl, 2-methoxy-4-chlorophenyl, 2-methoxy-4-fluorophenyl, 2-, 3- or 4-phenoxyphenyl, 2-, 3-or 4-phenylphenyl (2-, 3- or 4-biphenylyl), 2-, 3- or 4-benzylphenyl, 2-, 3- or 4-benzyloxyphenyl, 2-, 3- or 4-(4-chloro- or 4-fluorobenzyloxy)phenyl, 2-, 3- or 4-aminophenyl, 2-, 3- or 4-(N,N-dimethylamino)phenyl, 2-, 3- or 4-hydroxyphenyl, 2-

, 3- or 4-carboxyphenyl, 2-, 3- or 4-(methoxycarbonyl)phenyl, 2-, 3- or 4-morpholinophenyl and the corresponding ring substituted benzyl and α-methyl benzyl groups.

In a further aspect, therefore, the invention provides a method as defined above wherein $R^1$ and $R^2$ are both halophenyl and preferablyone wherein $R^1$ and $R^2$, which may be the same or different, are each chlorophenyl or fluorophenyl. Furthermore in another aspect the invention provides a method as defined above wherein at least one phenyl ring also bears one or more alkoxy groups, and, for example, $R^1$ may be 2-chloro-4-methoxyphenyl when $R^2$ is 2-chloro, 2-fluoro, 4-chloro-or 4-fluoro-phenyl.

In a preferred aspect the invention provides a method as defined above wherein the compound is : 1-(1,2,4-triazol-1-yl)-2-(4-fluorophenyl)-2-(4-chlorophenyl)ethan-2-ol; 1-(1,2,4-triazol-1-yl)-2-(2-fluorophenyl)-2-(4-fluorophenyl)ethan-2-ol; 1-(1,2,4-triazol-1-yl)-2-(4-fluorophenyl)-2-(2,4-dichlorophenyl)ethan-2-ol; 1-(1,2,4-triazol-1-yl)-2,2-di(4-fluorophenyl)ethan-2-ol; 1-(1,2,4-triazol-1-yl)-2-(4-chlorophenyl)-2-(2-chlorophenyl)ethan-2-ol; 1-(1,2,4-triazol-1-yl)-2(4-fluorophenyl)-2-(2-chlorophenyl)ethan-1-ol; 2,2-dimethyl-3-(2-methoxybenzyl)-4-(1,2,4-triazol-1-yl)-butan-3-ol; 1-(1,2,4-triazol-1-yl)-2-(2-chloro-4-methoxyphenyl)-2-(4-fluorophenyl)ethan-2-ol; 1-(1,2,4-triazol-1-yl)-2-(4-chlorophenyl)-3,3-dimethyl-butan-2-ol; 1-(1,2,4-triazol-1-yl)-bis-2-(4-fluorophenyl)-2-methoxy-ethane;1-(1,2,4-triazol-1-yl)-2-(4-fluorophenyl)-2-(2-fluoro-4-methyl-phenyl)ethan-2-ol; 1-(1,2,4-triazol-1-yl)-2-(4-fluorophenyl)-2-(2-chloro-4-methyl-phenyl)ethan-2-ol;

The salts of the compounds of the method and of the novel compounds can be salts with inorganic or organic acids e.g. hydrochloric, nitric, sulphuric, acetic, p-toluenesulphonic or oxalic acid.

Suitably the metal complex is one including, as the metal, copper, zinc, manganese or iron. It preferably has the general formula :

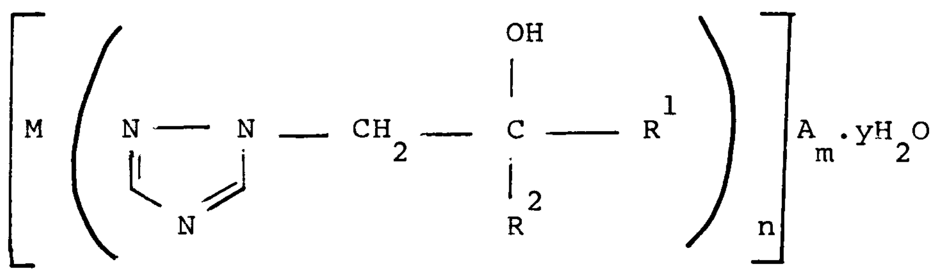

wherein Y, $R^1$ and $R^2$ are as defined above, M is a metal, A is an anion (e.g. a chloride, bromide, iodide, nitrate, sulphate or phosphate anion), n is 2 or 4, y is O or an integer of 1 to 12, and m is an integer consistent with valency.

Examples of the compounds of the invention are shown in Table I.

TABLE I

| Compound No. | $R^1$ | $R^2$ | Melting Point (°C) |
|---|---|---|---|
| 1 | $C_6H_5$- | $C_6H_5CH_2$- | 124-125 |
| 2 | $C_6H_5$- | p-Cl-$C_6H_4CH_2$- | 144-145 |
| 3 | $C_6H_5$- | p-F-$C_6H_4CH_2$- | 116-118 |
| 4 | p-Cl-$C_6H_4$- | p-Cl-$C_6H_4CH_2$- | 80-83 |
| 5 | p-Cl-$C_6H_4$- | $C_6H_5CH_2$- | 109-111 |
| 6 | p-F-$C_6H_4$- | $C_6H_5CH_2$- | 141-142 |
| 7* | $C_6H_5$- | 2,4-diCl-$C_6H_3CH_2$- | 104-106 |
| 8 | p-F-$C_6H_4$- | p-F-$C_6H_4CH_2$- | 154-156 |
| 9 | p-F-$C_6H_4$- | p-Cl-$C_6H_4CH_2$- | 168-170 |
| 10 | t-Bu | $C_6H_5CH_2$- | 110-111 |
| 11 | t-Bu | p-Cl-$C_6H_4CH_2$- | 86-87 |
| 12 | t-Bu | p-F-$C_6H_4CH_2$- | 146-148 |
| 13 | $C_6H_5$- | o-F-$C_6H_4CH_2$- | 133-134 |
| 14 | p-Cl-$C_6H_4$ | o-F-$C_6H_4CH_2$- | 95-96 |
| 15 | $C_6H_5$- | o-Cl-$C_6H_4CH_2$- | 69-71 |
| 16 | p-MeO-$C_6H_4$- | $C_6H_5CH_2$- | 100-103 |
| 17 | $C_6H_5$- | $C_6H_5$- | 128-129 |
| 18+ | p-F-$C_6H_4$- | p-F-$C_6H_4CH_2$- | 161-163 |
| 19 | $C_6H_5$- | 2,4-diCl-$C_6H_3CH_2$- | 104-106 |

* Includes 1 mole of $C_2H_5OH$

\+ This compound is a different crystalline form of compound No. 8

TABLE I (cont)

| Compound No. | $R^1$ | $R^2$ | Melting Point (°C) |
|---|---|---|---|
| 20 | t-Bu | o-Cl-$C_6H_4CH_2$- | 74-75 |
| 21 | t-Bu | o-F-$C_6H_4CH_2$- | 96-98 |
| 22 | t-Bu | m-Cl-$C_6H_4CH_2$- | 88-89 |
| 23 | t-Bu | m-$CF_3$-$C_6H_4CH_2$- | 106-107 |
| 24 | $C_6H_5$- | p-t-Bu-$C_6H_4CH_2$- | 80-83 |
| 25 | p-Cl-$C_6H_4$- | $C_6H_5$- | 83-85 |
| 26 | p-Cl-$C_6H_4$- | p-Cl-$C_6H_4$- | 147-148 |
| 27 | p-Cl-$C_6H_4$- | p-F-$C_6H_4$- | 154-155 |
| 28 | 2,4-diCl-$C_6H_3$- | $C_6H_5$- | 191-194 |
| 29 | p-F-$C_6H_4$- | p-F-$C_6H_4$- | 170-171 |
| 30 | p-F-$C_6H_4$- | $C_6H_5$- | 139-140 |
| 31 | i-Bu | $C_6H_5$- | 94-95 |
| 32 | n-Bu | p-Cl-$C_6H_5$- | 95-97 |
| 33 | t-Bu | 2-Cl-6-F-$C_6H_3CH_2$- | |
| 34 | t-Bu | 2-Cl-4-F-$C_6H_3CH_2$- | 95 |
| 35 | t-Bu | 2-F-4-Cl-$C_6H_3CH_2$- | 104-106 |
| 36 | t-Bu | 2,4-diCl-$C_6H$ | |
| 37 | t-Bu | 2,6-diCl-$C_6H_3CH_2$- | |
| 38 | t-Bu | 2,6-diF-$C_6H_3CH_2$- | |
| 39 | t-Bu | $C_6H_5$- | glass |

TABLE I (cont)

| Compound No. | $R^1$ | $R^2$ | Melting Point (°C) |
|---|---|---|---|
| 40 | $C_6H_5$- | p-t-Bu-$C_6H_4$- | 131-135° |
| 41 | $C_6H_5$- | o-Cl$C_6H_4$- | 142-143° |
| 42 | $C_6H_5$- | o-F-$C_6H_4$- | 126-128° |
| 43 | p-Cl-$C_6H_4$- | o-Cl-$C_6H_4$- | 137-138° |
| 44 | p-Cl-$C_6H_4$- | o-F-$C_6H_4$- | 144-145° |
| 45 | p-F-$C_6H_4$- | 0-Cl-$C_6H_4$- | 115-116° |
| 46 | p-F-$C_6H_4$- | o-F-$C_6H_4$- | 120-123° |
| 47 | $C_6H_5$- | o-$C_6H_5O$-$C_6H_4$- | |
| 48 | p-Cl-$C_6H_4$- | o-$C_6H_5O$-$C_6H_4$- | |
| 49 | $C_6H_5$- | o-Me-$C_6H_4$- | 161-162° |
| 50 | p-Cl-$C_6H_4$- | o-Me-$C_6H_4$- | 157-158° |
| 51 | 2,4-diCl-$C_6H_3$- | p-F-$C_5H_4$- | 137-138° |
| 52 | o-Cl-$C_6H_4$- | p-MeO-$C_6H_4$- | 184-185° |
| 53 | 2,4-diCl-$C_6H_3$- | p-Cl-$C_6H_4$- | 174-175° |
| 54 | 2,4-diCl-$C_6H_3$- | o-Cl-$C_6H_4$- | 149-151° |
| 55 | 2,4-diCl-$C_6H_3$- | o-F-$C_6H_4$ | 146-147° |
| 56 | p-$C_6H_5CH_2O$-$C_6H_4$- | $C_6H_5$- | 134-135° |
| 57 | p-(p-Cl-$C_6H_4CH_2O$)-$C_6H_4$- | $C_6H_5$- | 98-100° |
| 58 | m-Cl-$C_6H_4$- | p-Cl-$C_6H_4$- | 139-142° |
| 59 | p-(p-F-$C_6H_4CH_2O$)-$C_6H_4$- | $C_6H_5$- | 105-107° |

TABLE I (cont)

| Compound No. | $R^1$ | $R^2$ | Melting Point (°C) |
|---|---|---|---|
| 60 | m-Cl-$C_6H_4$- | p-F-$C_6H_4$- | 190-193° |
| 61 | m-Cl-$C_6H_4$- | p-MeO-$C_6H_4$- | 58-60° |
| 62 | 2,4-diCl-$C_6H_3$- | m-Cl-$C_6H_4$- | 139-142° |
| 63 | o-Me-$C_6H_4$- | p-F-$C_6H_4$- | 200-201° |
| 64 | p-F-$C_6H_4$- | p-$CO_2CH_3$-$C_6H_4$- | 164-166° (HCl salt) |
| 65 | p-$OC_2H_5$-$C_6H_4$- | p-Cl-$C_6H_4$- | 126-127° |
| 66 | p-$OCF_2H$-$C_6H_4$- | p-Cl-$C_6H_4$- | glass |
| 67 | p-$OCF_2H$-$C_6H_4$- | o-Cl-$C_6H_4$- | glass |
| 68 | o-Br-$C_6H_4$- | $C_6H_5$- | |
| 69 | o-Br-$C_6H_4$- | p-Cl-$C_6H_4$- | 151-152° |
| 70 | o-Br-$C_6H_4$- | p-F-$C_6H_4$- | 109-111° |
| 71 | p-$NO_2$-$C_6H_4$- | $C_6H_5$- | 164-166° |
| 72 | 2-Cl-5-$NO_2$-$C_6H_4$- | $C_6H_5$- | 206-208° |
| 73 | 2-Cl-4-$NO_2$-$C_6H_4$- | p-Cl-$C_6H_4$- | |
| 74 | o-$NH_2$-$C_6H_4$- | $C_6H_5$- | ° |
| 76 | o-$CO_2H$-$C_6H_4$- | p-F-$C_6H_4$- | |
| 77 | o-$CO_2Me$-$C_6H_4$- | p-F-$C_6H_4$- | |
| 78 | o-$CO_2H$-$C_6H_4$- | $C_6H_5$- | |
| 79 | o-$CO_2Me$-$C_6H_4$- | $C_6H_5$- | |
| 80 | o-OH-$C_6H_4$- | $C_6H_5$- | |
| 81 | o-MeO-$C_6H_4$- | $C_6H_5$- | 113-116° |
| 82 | o-MeO-$C_6H_4$- | p-Cl-$C_6H_4$- | 129-131° |

TABLE I (cont)

| Compound No. | $R^1$ | $R^2$ | Melting Point (°C) |
|---|---|---|---|
| 83 | o-MeO-$C_6H_4$- | p-F-$C_6H_4$- | 133-135° |
| 84 | p-OH-$C_6H_4$- | $C_6H_5$- | |
| 85 | p-Br-$C_6H_4$- | $C_6H_5$- | 104-105° |
| 86 | p-Br-$C_6H_4$- | p-Br-$C_6H_4$- | 159-160° |
| 87 | p-Br-$C_6H_4$- | p-Cl-$C_6H_4$- | 138-139° |
| 88 | p-$(CH_3)_2N$-$C_6H_4$- | $C_6H_5$- | |
| 89 | o-Me-$C_6H_4$- | p-Me-$C_6H_4$- | 117-120° |
| 90 | o-Cl-$C_6H_4$- | p-Me-$C_6H_4$- | 169-170° |
| 91 | o-F-$C_6H_4$- | p-Me-$C_6H_4$- | 156-158° |
| 92 | O-Br-$C_6H_4$- | p-Me-$C_6H_4$- | |
| 93 | o-$NH_2$-$C_6H_4$- | p-Cl-$C_6H_4$- | |
| 94 | o-$CO_2H$-$C_6H_4$- | p-Me-$C_6H_4$- | |
| 95 | o-$CO_2Me$-$C_6H_4$- | p-Me-$C_6H_4$- | |
| 96 | p-MO-$C_6H_4$- | $C_6H_5$- | |
| 97 | p-F-$C_6H_4$- | p-$NO_2$-$C_6H_4$- | 145-148° |
| 98 | o-$CO_2H$-$C_6H_4$- | p-Br-$C_6H_4$- | |
| 99 | o-$CO_2Me$-$C_6H_4$- | p-Br-$C_6H_4$- | |
| 100 | o-$CO_2H$-$C_6H_4$- | p-Cl-$C_6H_4$- | |
| 101 | o-$CO_2Me$-$C_6H_4$- | p-Cl-$C_6H_4$- | |
| 102 | p-Me-$C_6H_4$- | p-Me-$C_6H_4$- | 153-154° |
| 103 | 2,4-diMe-$C_6H_4$- | $C_6H_5$- | 148-149° |

TABLE I (cont)

| Compound No. | $R^1$ | $R^2$ | Melting Point (°C) |
|---|---|---|---|
| 104 | m-$NO_2$-$C_6H_4$- | $C_6H_5$- | 212-215° (HCl salt) |
| 105 | o-Cl-$C_6H_4$- | o-Cl-$C_6H_4$- | 164-166° |
| 106 | o-$CF_3$-$C_6H_4$- | $C_6H_5$- | |
| 107+ | m-$CF_3$-$C_6H_4$- | $C_6H_5$- | 115-117° |
| 108 | p-$CF_3$-$C_6H_4$- | $C_6H_5$- | 207-210° (HCl salt) |
| 109 | m-$CF_3$-$C_6H_4$- | p-Cl-$C_6H_4$- | 104-106° |
| 110 | p-$OCF_3$-$C_6H_4$- | $C_6H_5$- | glass |
| 111 | p-$OCF_3$-$C_6H_4$- | p-Cl-$C_6H_4$- | 107-108° |
| 112 | p-$OCF_3$-$C_6H_4$- | o-Cl-$C_6H_4$- | 99-100° |
| 113 | p-$OCF_3$-$C_6H_4$- | p-$OCF_3$-$C_6H_4$- | |
| 114 | m-$OCF_3$-$C_6H_4$- | $C_6H_5$- | |
| 115 | m-$OCF_3$-$C_6H_4$- | p-Cl-$C_6H_4$- | |
| 116 | m-$OCF_3$-$C_6H_4$- | p-Br-$C_6H_4$- | |
| 117 | o-$OCF_2CHF_2$-$C_6H_4$- | $C_6H_5$- | 188-189° |
| 118 | o-$OCF_2CHF_2$-$C_6H_4$- | p-Cl-$C_6H_4$- | 94-95° |
| 119 | m-$OCF_2CHF_2$-$C_6H_4$- | $C_6H_5$- | |
| 120 | m-$OCF_2CHF_2$-$C_6H_4$- | p-Cl-$C_6H_4$- | |
| 121 | p-$OCF_2CHF_2$-$C_6H_4$- | $C_6H_5$- | glass |
| 122* | p-$OCF_2CHF_2$-$C_6H_4$- | p-Cl-$C_6H_4$- | glass |
| 123 | p-$OCF_2CHF_2$-$C_6H_4$- | p-$OCF_2CHF_2$-$C_6H_4$- | glass |
| 124 | 3,4-Z-$C_6H_4$- | $C_6H_5$- | |

+ melting point of HCl salt of this compound is 184-185°

* melting point of the 1:1 complex of this compound with isopropyl alcohol is 74.5-77.5°.

TABLE I (cont)

| Compound No. | $R^1$ | $R^2$ | Melting Point (°C) |
|---|---|---|---|
| 125 | 3,4-Z-$C_6H_4$- | p-Cl-$C_6H_4$- | glass |
| 126 | 3,4-Z-$C_6H_4$- | o-Cl-$C_6H_4$- | |
| 127 | 3,4-Z-$C_6H_4$- | p-F-$C_6H_4$- | |
| 128 | t-Bu | o-Br-$C_6H_4$-$CH_2$- | 111-115° |
| 129 | t-Bu | 2,4-diF-$C_6H_3$-$CH_2$- | 140° |
| 130 | t-Bu | o-MeO-$C_6H_4$-$CH_2$- | 113-116° |
| 131 | Me | $C_6H_5$- | oil |
| 132 | Me | p-Cl-$C_6H_4$- | 88-90° |
| 133 | Me | 2,4-diCl-$C_6H_4$- | 77-81° |
| 134 | Me | p-F-$C_6H_4$- | |
| 135 | Me | o-Cl-$C_6H_4$- | |
| 136 | Me | o-F-$C_6H_4$- | |
| 137 | Me | o-Me-$C_6H_4$- | |
| 138 | Me | 2,4-diMe-$C_6H_3$- | |
| 139 | Et | $C_6H_5$- | 96-97° |
| 140 | Et | p-Cl-$C_6H_4$- | 105-106° |
| 141 | Et | 2,4-diCl-$C_6H_4$- | |
| 142 | Et | p-F-$C_6H_4$- | 94° |
| 143 | Et | o-Cl-$C_6H_4$- | |
| 144 | Et | o-F-$C_6H_4$- | |
| 145 | Me | o-Me-$C_6H_4$- | |

TABLE I (cont)

| Compound No. | $R^1$ | $R^2$ | Melting Point (°C) |
|---|---|---|---|
| 146 | Me | 2,4-diMe-$C_6H_3$- | |
| 147 | n-Pr | $C_6H_5$- | 77-79° |
| 148 | n-Pr | p-Cl-$C_6H_4$- | |
| 149 | n-Pr | p-F-$C_6H_4$- | |
| 150 | n-Pr | 2,4-diCl-$C_6H_3$- | |
| 151 | n-Pr | o-Cl-$C_6H_4$- | |
| 152 | n-Pr | o-F-$C_6H_4$- | |
| 153 | n-Pr | o-Me-$C_6H_4$- | |
| 154 | n-Pr | 2,4-diMe-$C_6H_3$- | |
| 155 | n-Pr | p-MeO-$C_6H_4$- | |
| 156 | n-Pr | O-MeO-$C_6H_4$- | |
| 157 | i-Pr | $C_6H_5$- | |
| 158 | i-Pr | p-Cl-$C_6H_4$- | |
| 159 | i-Pr | p-F-$C_6H_4$- | |
| 160 | i-Pr | 2,4-diCl-$C_6H_3$- | |
| 161 | i-Pr | o-Cl-$C_6H_4$- | |
| 162 | i-Pr | o-F-$C_6H_4$- | |
| 163 | i-Pr | o-Me-$C_6H_4$- | |
| 164 | i-Pr | 2,4-diMe-$C_6H_4$- | |
| 165 | i-Pr | O-MeO-$C_6H_4$- | |
| 166 | i-Pr | p-MeO-$C_6H_4$- | |

TABLE I (cont)

| Compound No. | $R^1$ | $R^2$ | Melting Point (°C) |
|---|---|---|---|
| 167 | $C_3H_5$- | $C_6H_5$- | 91-92° |
| 168 | $C_3H_5$- | p-Cl-$C_6H_4$- | oil |
| 169 | $C_3H_5$- | p-F-$C_6H_4$- | |
| 170 | $C_3H_5$- | 2,4-diCl-$C_6H_3$- | |
| 171 | $C_3H_5$- | o-Cl-$C_6H_4$- | |
| 172 | $C_3H_5$- | o-F-$C_6H_4$- | |
| 173 | $C_3H_5$ | o-Me-$C_6H_4$- | |
| 174 | $C_3H_5$ | 2,4-diMe-$C_6H_3$- | |
| 175 | $C_3H_5$ | o-MeO-$C_6H_4$- | |
| 176 | $C_3H_5$ | p-MeO-$C_6H_4$- | 101-102° |
| 177 | n-Bu | $C_6H_5$- | 62-63° |
| 178 | n-Bu | p-F-$C_6H_4$- | 93-95° |
| 179 | n-Bu | 2,4-diCl-$C_6H_3$- | 106-108° |
| 180 | n-Bu | o-Cl-$C_6H_4$- | |
| 181 | n-Bu | o-F-$C_6H_4$- | |
| 182 | n-Bu | o-Me-$C_6H_4$- | |
| 183 | n-Bu | 2,4-diMe-$C_6H_3$- | |
| 184 | n-Bu | o-MeO-$C_6H_4$- | |
| 185 | n-Bu | p-MeO-$C_6H_4$- | |
| 186 | i-Bu | p-Cl-$C_6H_4$- | |
| 187 | i-Bu | p-F-$C_6H_4$- | |

TABLE I (cont)

| Compound No. | $R^1$ | $R^2$ | Melting Point (°C) |
|---|---|---|---|
| 188 | i-Bu | 2,4-diCl-$C_6H_3$- | |
| 189 | i-Bu | o-Cl-$C_6H_4$- | |
| 190 | i-Bu | o-F-$C_6H_4$- | |
| 191 | i-Bu | o-Me-$C_6H_4$- | |
| 192 | i-Bu | 2,4-diMe-$C_6H_3$- | |
| 193 | i-Bu | p-MeO-$C_6H_4$- | |
| 194 | i-Bu | oMeO-$C_6H_4$- | |
| 195 | t-Bu | $C_6H_5$- | 75° |
| 196 | t-Bu | p-Cl-$C_6H_4$- | 70-73° |
| 197 | t-Bu | 2,4-diCl-$C_6H_3$- | |
| 198 | t-Bu | p-F-$C_6H_4$- | 92-93° |
| 199 | t-Bu | o-Cl-$C_6H_4$- | |
| 200 | t-Bu | o-F-$C_6H_4$- | |
| 201 | t-Bu | o-Me-$C_6H_4$- | |
| 202 | t-Bu | 2,4-diMe-$C_6H_3$- | |
| 203 | t-Bu | o-MeO-$C_6H_4$- | |
| 204 | t-Bu | p-MeO-$C_6H_4$- | 48° |
| 205 | n-Pe | $C_6H_5$- | |
| 206 | n-Pe | p-Cl-$C_6H_4$- | |
| 207 | n-Pe | 2,4-diCl-$C_6H_3$- | |
| 208 | n-Pe | p-F-$C_6H_4$- | |

TABLE I (cont)

| Compound No. | $R^1$ | $R^2$ | Melting Point (°C) |
|---|---|---|---|
| 209 | n-Pe | o-Cl-$C_6H_4$- | |
| 210 | n-Pe | o-F-$C_6H_4$- | |
| 211 | n-Pe | o-Me-$C_6H_4$- | |
| 212 | n-Pe | 2,4-diMe-$C_6H_3$- | |
| 213 | n-Pe | o-MeO-$C_6H_4$- | |
| 214 | n-Pe | p-MeO-$C_6H_4$- | |
| 215 | n-Pe | $C_6H_5$ | |
| 216 | $C_5H_9$- | p-Cl-$C_6H_4$- | |
| 217 | $C_5H_9$- | 2,4-diCl-$C_6H_4$- | |
| 218 | $C_5H_9$ | p-F-$C_6H_4$- | |
| 219 | $C_5H_9$- | o-Cl-$C_6H_4$- | |
| 220 | $C_5H_9$ | o-F-$C_6H_4$- | |
| 221 | $C_5H_9$ | o-Me-$C_6H_4$- | |
| 222 | $C_5H_9$ | p-Me-$C_6H_4$- | |
| 223 | $C_5H_9$ | o-MeO-$C_6H_4$- | |
| 224 | $C_5H_9$ | P_MeO-$C_6H_4$- | |
| 225 | n-He | $C_6H_5$- | |
| 226 | n-He | p-Cl-$C_6H_4$- | |
| 227 | n-He | 2,4-diCl-$C_6H_3$- | |
| 228 | n-He | p-F-$C_6H_4$- | 97-99° |

TABLE I (cont)

| Compound No. | $R^1$ | $R^2$ | Melting Point (°C) |
|---|---|---|---|
| 229 | n-He | o-Cl-$C_6H_4$- | |
| 230 | n-He | o-F-$C_6H_4$- | |
| 231 | n-He | o-Me-$C_6H_4$- | |
| 232 | n-He | 2,4-diMe-$C_6H_3$- | |
| 233 | n-He | o-MeO-$C_6H_4$- | |
| 234 | n-He | p-MeO-$C_6H_4$- | |
| 235 | $C_6H_{11}$ | $C_6H_5$- | 110-111° |
| 236 | $C_6H_{11}$ | p-Cl-$C_6H_4$- | 64° |
| 237 | $C_6H_{11}$ | 2,4-diCl-$C_6H_4$- | |
| 238 | $C_6H_{11}$ | p-F-$C_6H_4$- | |
| 239 | $C_6H_{11}$ | o-Cl-$C_6H_4$- | |
| 240 | $C_6H_{11}$ | o-F-$C_6H_4$- | |
| 241 | $C_6H_{11}$ | O-Me-$C_6H_4$- | |
| 242 | $C_6H_{11}$ | 2,4-diMe-$C_6H_3$- | |
| 243 | $C_6H_{11}$ | o-MeO-$C_6H_4$- | |
| 244 | $C_6H_{11}$ | p-MeO-$C_6H_4$- | 92-94° |
| 245 | t-Bu | 2-F-4-MeO-$C_6$H3$CH_2$- | |
| 246 | t-Bu | 2-MeO-4-F-$C_6H_3CH_2$- | |
| 247 | t-Bu | 2-MeO-4-Cl-$C_6H_3CH_2$- | |
| 248 | t-Bu | p-MeO-$C_6H_4CH_2$- | 130-131° |
| 249 | t-Bu | p-$CF_3$-$C_6H_4CH_2$ | |

TABLE I (cont)

| Compound No. | $R^1$ | $R^2$ | Melting Point (°C) |
|---|---|---|---|
| 250 | $3-NO_2 4-ClC_6H_3-$ | $C_6H_5-$ | 139-141° |
| 251 | $m-Cl-C_6H_4-$ | $C_6H_5-$ | 108-110° |
| 252 | $m-F-C_6H_4-$ | $p-Cl-C_6H_4-$ | 55-59° |
| 253 | $m-F-C_6H_4-$ | $p-F-C_6H_4-$ | 116-118° |
| 254 | $p-OCH_3C_6H_4-$ | $C_6H_5-$ | 88-89° |
| 255 | $p-OC_6H_5-C_6H_4-$ | $C_6H_5-$ | 103-105° |
| 256 | $o-OCH_3-C_6H_4-$ | $p-OCH_3-C_6H_4-$ | 133-134° |
| 257 | $o-F-C_6H_4-$ | $p-OCH_3-C_6H_4-$ | 144-145° |
| 258 | $p-NO_2-C_6H_4-$ | $p-Cl-C_6H_4-$ | 157-158° |
| 259 | $p-OCH_3-C_6H_4-$ | $p-Cl-C_6H_4-$ | 105-106° |
| 260 | $p-OC_6H_5-C_6H_4-$ | $o-Cl-C_6H_4-$ | 100-102° |
| 261 | $p-OC_6H_5-C_6H_4-$ | $o-F-C_6H_4-$ | 132-133° |
| 262 | $o,p-diCl-C_6H_3-$ | $p-OCH_3-C_6H_4-$ | 178-179° |
| 263 | $p-OCH_3-C_6H_4-$ | $p-F-C_6H_4-$ | 124-125° |
| 264 | $p-OCF_3CHF_2-C_6H_4-$ | $o,p-diCl-C_6H_4-$ | 125-126° |
| 265 | $p-CO_2Me-C_6H_4-$ | $C_6H_5-$ | 70-72° |
| 266 | $p-CN-C_6H_4-$ | $C_6H_5-$ | 82-85° |
| 267 | $p-CH_3-C_6H_4-$ | $p-Cl-C_6H_4-$ | 134-135° |
| 268 | $p-CH_3-C_6H_4-$ | $p-F-C_6H_4-$ | 124-125° |

TABLE I (cont)

| Compound No. | $R^1$ | $R^2$ | Melting Point (°C) |
|---|---|---|---|
| 269 | o-$OCH_3$-$C_6H_4$- | 2,4-diCl-$C_6H_3$- | 152-153° |
| 270 | p-$OC_6H_5$-$C_6H_4$- | p-Cl-$C_6H_4$- | gum HCl salt m.p. 177-178° |
| 272 | p-$OC_6H_5$-$C_6H_4$- | p-F-$C_6H_4$- | 138-140° HCl salt m.p. 171-173° |
| 272 | p-$OCH(CH_3)_2$-$C_6H_4$- | p-Cl-$C_6H_4$- | 151-153° |
| 273 | n-Bu | p-$CH_3$-$C_6H_4$- | 80-81° |
| 274 | n-He | p-$CH_3$-$C_6H_4$- | 76-78° |
| 275 | 4-$OCF_3$-$C_6H_4$- | 2-F-$C_6H_4$- | glass |
| 276 | ditto | 4-F-$C_6H_4$- | 132-133° |
| 277 | ditto | 2-$OCH_3$-$C_6H_4$- | 115-116° |
| 278 | 2-$OCF_3H$-$C_6H_4$- | 2-Cl-$C_6H_4$- | 114-115° |
| 279 | 2-$OCF_2CF_2H$-$C_6H_4$- | 4-F-$C_6H_4$- | 115-116° |
| 280 | 4-$OCF_2CF_2H$-$C_6H_4$- | ditto | 125-126° |
| 281 | ditto | 2-F-$C_6H_4$- | 110-111° |

TABLE I (cont)

| Compound No. | $R^1$ | $R^2$ | Melting Point (°C) |
|---|---|---|---|
| 282 | O<br>$CH_2$<br>O | 4-Cl-$C_6H_4$- | 129-130° |
| 283 | 2,4-di-Cl-$C_6H_3$- | <u>n</u>-$C_3H_7(CH_3)CH.CH_2$- | Diastereoisomer A<br>98-99°<br>Diastereoisomer B<br>93-94° |
| 284 | 2-Cl-$C_6H_4$- | 4-$OC_2H_5$-$C_6H_4$- | 94-96° |
| 285 | 2,4-diCl-$C_6H_4$- | 4-$OC_2H_5$-$C_6H_4$- | 159-161° |
| 286 | 4-Cl-$C_6H_4$- | 4-$NH_2$-$C_6H_4$- | 56° |
| 287 | 4-Cl-$C_6H_4$- | 4-$CH_3CONH$-$C_6H_4$- | 70-75° |
| 288 | 4-Cl-$C_6H_4$- | 4-CN-$C_6H_4$- | 160° |
| 289 | 4-F-$C_6H_4$- | 4-CN-$C_6H_4$- | 139-140° |
| 290 | 4-$CH_3$-$C_6H_4$- | 2,4-diCl-$C_6H_3$- | 183° |
| 291 | 2-Cl-$C_6H_4$- | 2-$OCH_3$-$C_6H_4$- | 148-150° |
| 292 | 4-CN-$C_6H_4$- | 2,4-diCl-$C_6H_3$- | 182-184 |
| 293 | 2-F-4-$OCH_3$-$C_6H_3$- | 4-F-$C_6H_4$- | 161-162° |
| 294 | 2-Cl-4-$OCH_3$-$C_6H_3$- | 4-F-$C_6H_4$- | 138-140° |
| 295 | 2-Cl-4-$OCH_3$-$C_6H_3$- | 4-Cl-$C_6H_4$- | 176-177° |
| 296 | 2-F-4-$OCH_3$-$C_6H_3$- | 2-Cl-$C_6H_4$- | 170-172° |
| 297 | 2-F-4-$OCH_3$-$C_6H_3$- | 4-Cl-$C_6H_4$- | 134-136° |

TABLE I (cont)

| Compound No. | $R^1$ | $R^2$ | Melting Point (°C) |
|---|---|---|---|
| 298 | $2\text{-Cl-4-CH}_3\text{-C}_6\text{H}_3\text{-}$ | $4\text{-F-C}_6\text{H}_4\text{-}$ | 158° |
| 299 | $2\text{-CH}_3\text{-4-Cl-C}_6\text{H}_3\text{-}$ | $4\text{-F-C}_6\text{H}_4\text{-}$ | 65-67° |
| 300 | $2\text{-F-4-CH}_3\text{-C}_6\text{H}_3\text{-}$ | $4\text{-F-C}_6\text{H}_4\text{-}$ | - |
| 301 | $C_4H_7$ | $C_6H_5$ | oil |
| 302 | $4\text{-NO}_2\text{-C}_6\text{H}_4\text{-}$ | $4\text{-NO}_2\text{-C}_6\text{H}_4\text{-}$ | |
| 303 | $4\text{-NH}_2\text{-C}_6\text{H}_4\text{-}$ | $4\text{-NH}_2\text{-C}_6\text{H}_4\text{-}$ | |
| 304 | s-butyl | $2,4\text{-diCl}_2\text{-C}_6\text{H}_3\text{-}$ | |
| 305 | s-butyl | $4\text{-Cl-C}_6\text{H}_4\text{-}$ | |
| 306 | $4\text{-Cl-C}_6\text{H}_4\text{-}$ | $3\text{-CH}_3\text{-4-ClC}_6\text{H}_5\text{-}$ | 175-176° |
| 307 | $2,4\text{-diCl-C}_6\text{H}_3\text{-}$ | $2,4\text{-diCl-C}_6\text{H}_3\text{-}$ | 170° |
| 308 | $2\text{-NO}_2\text{-C}_6\text{H}_4\text{-}$ | $3\text{-NO}_2\text{-C}_6\text{H}_4\text{-}$ | 170-172° |
| 309 | $4\text{-C}_2\text{H}_5\text{O-C}_6\text{H}_4\text{-}$ | $4\text{-C}_2\text{H}_5\text{O-C}_6\text{H}_4\text{-}$ | gum |

* Includes 1 mole of ethanol occluded in the crystal lattice.

\+ Compounds 8 and 18 were obtained as polymorphs and this explains their different melting points.

n-Pe = n-Pentyl
n-He = n-hexyl
$C_3H_5$ = cyclopropyl
$C_4H_7$ = cyclobutyl
$C_5H_9$ = cyclopentyl
$C_6H_{11}$ = cyclohexyl
MO = morpholino
Z = difluoromethylenedioxy

The compounds of general formula (I) may be produced by reacting a compound of general formula (II) or (III):

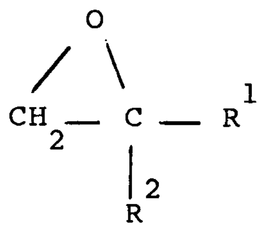

(II)

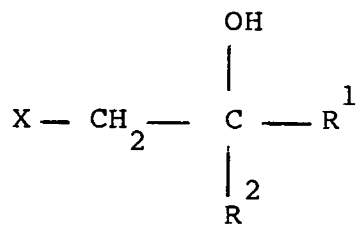

(III)

in which $R^1$ and $R^2$ are as defined above and X is a halogen atom (preferably a chlorine or bromine atom), with 1,2,4-triazole either in the presence of an acid-binding agent or in the form of one of its alkali metal salts in a convenient solvent. Suitably the compound of general formula (II) or (III) is reacted at 20-100 `C with the sodium salt of 1,2,4-triazole (the salt can be prepared by adding either sodium hydride or sodium methoxide to 1,2,4-triazole) in a convenient solvent such as acetonitrile, methanol, ethanol or dimethylformamide. The product can be isolated by pouring the reaction mixture into water and recrystallising the solid formed from a convenient solvent.

The compounds of general formula (II) and (III) can be prepared by reacting a compound of general formula (IVa) or (IVb):

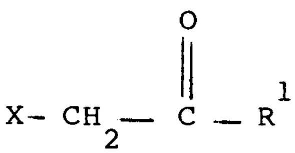

(IVa)

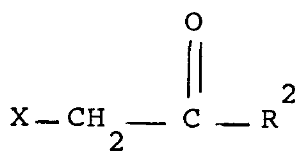

(IVb)

wherein $R^1$, $R^2$ and X are as defined above with, respectively, a Grignard compound of general formula (Va) or (Vb) :

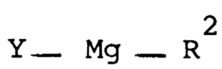

(Va)

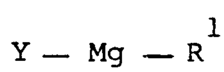

(Vb)

wherein $R^1$ and $R^2$ are as defined above and Y us a halogen (preferably chlorine, bromine or iodine) in a convenient solvent such as diethyl ether or tetrahydrofuran. Generally a mixture of the compounds of general formula (II) and (III) are obtained. For example, when a compound of general formula (IVa) wherein $R^1$ is alkyl or cycloalkyl is reacted, the compound of formula (II) generally predominates in the mixture; on the other hand, when $R^1$ is optionally substituted phenyl, the compound of general formula (III) generally predominates in the mixture.

The compounds of general formula (IV) and (V) may be made by methods set out in the literature.

The compounds of general formula (II) wherein each of $R^1$ and $R^2$, which may be the same or different, is substituted phenyl may also be prepared by reacting the appropriate benzophenone compound of general formula (VI)

$R^1— CO— R^2$

wherein $R^1$ and $R^2$ are as defined above, with dimethyl oxosulphonium methylide (Corey and Chaykovsky JACS, 1965, 87, 1353-1364) or dimethyl sulphonium methylide (Corey and Chaykovsky, JACS, 1962, 84,

3782) using methods set out in the literature.

The benzophenone compounds of general formula (VI) can be prepared, using the Friedel-Crafts reaction, by reacting a substituted benzoyl chloride with the appropriately substituted benzene in the presence of a Lewis acid e.g. aluminium chloride.

The compounds of general formula (II) wherein each of $R^1$ is alkyl, cycloalkyl or optionally substituted phenyl and $R^2$ is optionally substituted phenyl or optionally substituted benzyl can also be produced by reacting a $\beta$-hydroxy selenide compound of general formula (VII)

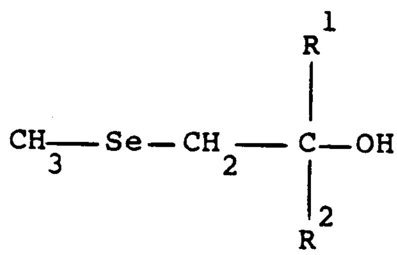

(VII)

wherein $R^1$ and $R^2$ are as defined above, with methyl iodide in potassium t-butoxide according to the method of Van Ende, Dumont and Krief, Angew. Chem. Int. Ed., 1975, 14, 700.

The $\beta$-hydroxy selenide compound can be prepared by treating the diselenide with the appropriate ketone in the presence of butyl lithium.

The compounds of general formula (III) wherein $R^1$ is alkyl or cycloalkyl and $R^2$ is optionally substituted benzyl (particularly benzyl ring substituted with alkoxy) can also be prepared by reacting a compound of general formula (VIII)

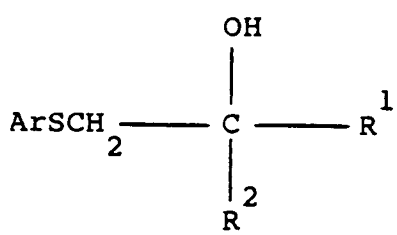

(VIII)

wherein $R^1$ and $R^2$ are as defined above and Ar is aryl (e.g. phenyl) with an alkylating agent to give the corresponding sulphonium salt which is then reacted with 1,2,4-triazole in the form of an alkali metal (e.g. sodium or potassium) salt. The compound of general formula (VIII) can be prepared by methods known in the art.

The salts and metal complexes of the compounds of general formula (I) can be prepared from the latter in known manner. For example, the complexes can be made by reacting the uncomplexed compound with a metal salt in a suitable solvent.

The compounds of general formula (I) are generally prepared by the above reactions in the form of racemic mixtures. The resolution of these mixtures into the constituent enantiomers can be performed by known methods. Examples of these methods are (1) forming the diastereoisomeric salts or esters of the compound of general formula (I) with an optically active acid (e.g. camphor sulphonic acid), separating the isomeric salts or esters and converting the separated isomeric salts or esters into the enantiomers of the compound of general formula (I); (2) forming the diastereoisomeric carbamates of the compound of general formula (I) by reacting a halo-formate (e.g. chloroformate) of the latter with an optically active amine (e.g. $\alpha$-methylbenzylamine), separating the isomeric carbamates, and converting the separated isomeric carbamates into the enantiomers of the compound of general formula (I), reacting the hemiphthate with an optically active amine (e.g. $\alpha$-methylbenzylamine) to give a salt of the hemiphthate, separating the isomeric salts and converting the separated salts into the enantiomers of the compound of general formula (I); or (4) resolving the mixtures using enantio-selective crystallisation techniques (Leigh, Chemistry and Industry,

1970, pages 1016-1017, and ibid, 1977, page 36). The separation of the diastereoisomeric salts, esters and carbamates can be achieved by for example crystallisation techniques or by high pressure liquid chromatography (HPLC). Alternatively, the enantiomers can be prepared directly from the compound of general formula (II) by stereospecific reduction, for example by biochemical reduction (using for example yeast or Aspergillus niger) or by hydrogenation using chiral catalysts (e.g. a Wilkinson's catalyst) or by reduction with borohydride/amino acid complexes.

In carrying out the method of the invention, the amount of compound to be applied to will depend upon a number of factors, for example the particular compound selected for use, and the identity of the material to be treated. Routine tests may be necessary to determine the best rate of application of a specific compound for any specific material for which it is suitable.

The compounds may be used as such but are more conveniently formulated into compositions for such usage.

The compounds can be applied in a number of ways, for example they can be applied formulated or unformulated, directly to the material or they can be sprayed on, dusted on or applied as a cream or paste formulation, or they can be applied as a vapour. Application can be to any part of the material and can be preventative, protectant, proplylactic and eradicant treatments.

The compounds are preferably used in the form of a composition. The type of composition used in any instance will depend upon the particular purpose envisaged.

The compositions may be in the form of dusting powders or granules comprising the active ingredient and a solid diluent or carrier, for example fillers such as kaolin, bentonite, kieselguhr, dolomite, calcium carbonate, talc, powdered magnesia, Fuller's earth, gypsum, Hewitt's earth, diatomaceous earth and China clay. Such granules can be preformed granules suitable for application to the soil without further treatment. These granules can be made either by impregnating pellets of filler with the active ingredient or by pelleting a mixture of the active ingredient and powdered filler. Compositions may comprise an agent (for example a mineral oil) for assisting the adhesion of the composition to the material; alternatively the active ingredient can be formulated for material dressing purposes using an organic solvent (for example N-methylpyrrolidone or dimethylformamide).

The compositions may also be in the form of dispersible powders, granules or grains comprising a wetting agent to facilitate the disperion in liquids of the powder or grains which may contain also fillers and suspending agents.

The aqueous dispersions or emulsions may be prepared by dissolving the active ingredient(s) in an organic solvent optionally containing wetting, dispersing or emulsifying agent(s) and then adding the mixture to water which may also contain wetting, dispersing or emulsifying agent(s). Suitable organic solvents are ethylene dichloride, isopropyl alcohol, propylene glycol, diacetone alcohol, toluene, kerosene, methylnaphthalene, the xylenes, trichloroethylene, furfuryl alcohol, tetrahydrofurfuryl alcohol, and glycol ethers (e.g. 2-ethoxyethanol and 2-butoxyethanol).

The compositions to be used as sprays may also be in the form of aerosols wherein the formulation is held in a container under pressure in the presence of a propellant, e.g. fluorotrichloromethane or dichlorodifluoromethane.

The compounds can be mixed in the dry state with a pyrotechnic mixture to form a composition suitable for generating in enclosed spaces a smoke containing the compounds.

Alternatively, the compounds may be used in a micro-encapsulated form.

By including suitable additives, for example additives for improving the distribution, adhesive power and resistance to rain on treated surfaces, the different compositions can be better adapted for various utilities.

The compositions may also be in the form of liquid preparations for use as dips or sprays which are generally aqueous dispersions or emulsions containing the active ingredient in the presence of one or more surfactants e.g. wetting agent(s), dispersing agent(s), emulsifying agent(s) or suspending agent(s). These agents can be cationic, anionic or non-anionic agents. Suitable cationic agents are quaternary ammonium compounds, for example cetyltrimethylammonium bromide.

Suitable anionic agents are soaps, salts of aliphatic monoesters of sulphuric acid (for example sodium lauryl sulphate), and salts of sulphonated aromatic compounds (for example sodium dodecylbenzenesulphonate, sodium, calcium or ammonium lignosulphonate, butylnaphthalene sulphonate, and a mixture of sodium diisopropyl- and triisopropyl-naphthalene sulphonates).

Suitable non-ionic agents are the condensation products of ethylene oxide with fatty alcohols such as oleyl or cetyl alcohol, or with alkyl phenols such as octyl- or nonyl-phenol and octylcresol. Other non-ionic agents are the partial esters derived from long chain fatty acids and hexitol anhydrides, the condensation products of the said partial esters with ethylene oxide, and the lecithins. Suitable suspending agents are hydrophilic colloids (for example polyvinylpyrrolidone and sodium carboxymethylcellulose), and the vegetable gums (for example gum acacia and gum tragacanth).

The compositions for use as aqueous dispersions or emulsions are generally supplied in the form of a concentrate containing a high proportion of the active ingredient(s), the concentrate to be diluted with water before use. These concentrates often should be able to withstand storage for prolonged periods and after such storage be capable of dilution with water in order to form aqueous preparations which remain homogenous for a sufficient time to enable them to be applied by conventional spray equipment. The concentrates may conveniently contain up to 95%, suitably 10-85%, for example 25-60%, by weight of the active ingredient(s). These concentrates suitably contain organic acids (e.g. alkaryl or aryl sulphonic acids such as xylene-sulphonic acid or dodecylbenzenesulphonic acid) since the presence of such acids can increase the solubility of the active ingredient(s) in the polar solvents often used in the concentrates. The concentrates suitably contain also a high proportion of surfactants so that sufficiently stable emulsions in water can be obtained. After dilution to form aqueous preparations, such preparations may contain varying amounts of the active ingredient(s) depending upon the intended purpose, but an aqueous preparation containing 0.0005% or 0.01% to 10% by weight of active ingredient(s) may be used.

The compositions of this invention can comprise also other compound(s) having biological activity, e.g. compounds having similar or complementary fungicidal or activity or compounds having insecticidal activity. Mixtures of fungicides can have a broader spectrum of activity than the compound of general formula (I) alone; further the other fungicide can have a synergistic effect on the fungicidal activity of the compound of general formula (I). Examples of the other fungicidal compound are imazalil, benomyl, carbendazim, thiophanate-methyl, captafol, captan, sulphur, triforine, dodemorph, tridemorph, pyrazophos, furalaxyl, ethirimol, dimethirimol, bupirimate, chlorothalonil, vinclozolin, procymidone, iprodione, metalaxyl, fosetyl-aluminium, carboxin, oxycarboxin, fenarimol, nuarimol, fenfuram, methfuroxam, nitrotal-isopropyl, triadimefon, thiabendazole, etridiazole, triadimenol, biloxazol, dithianon, binapacryl, quinomethionate, guazatine, dodine, fentin acetate, fentin hydroxide, dinocap, folpet, diclofluanid, ditalimphos, kitazin, fen-propemorph, cycloheximide, dichlobutrazol, a dithiocarbamate, a copper compound, a mercury compound, DPX 3217, RH 2161, Chevron RE 20615, CGA 64250, CGA 54251 and RO 14-3169.

Suitable insecticides are pirimor, croneton, dimethoate, metasystox and formothion.

The following Examples illustrate the preparation of compounds useful in invention methods; the temperatures are given in degrees Centigrade ( `).

EXAMPLE 1

This Example illustrates the preparation of :

1-(1,2,4-Triazol-1-yl)-2,3-diphenyl-propan-2-ol (Compound No 1 of Table I)

Benzyl chloride (0.2 mol) was dissolved in dry diethyl ether (200 ml) and added dropwise to magnesium turnings (0.22 g atoms). After all the magnesium had reacted, the solution was refluxed for 1 hour and cooled to room temperature. Phenacyl chloride (0.1 mol) in dry diethyl ether (100 ml) was added dropwise over 1 hour at such a rate as to maintain gentle reflux. The solution was then refluxed for 2 hours, and cooled to room temperature; the mixture was poured into ice and the complex decomposed with ammonium chloride solution. The ethereal solution was washed several times with water (2 x 200 ml), dried ($Na_2SO_4$), and the solvent removed in vacuo to give, as a colourless oil, the crude chlorohydrin which was dissolved in dimethyl formamide (80 ml) and a solution of sodium triazole [prepared from sodium (0.1 g atoms) in methanol (40 ml) and 1,2,4-triazole (0.1 mol)] added dropwise at room temperature. After stirring at room temperature for 2 hours, the solution was warmed at 50 ` for 3 hours. The solvent was removed in vacuo and the residue poured into water to give a crystalline solid which was recrystallised from ethanol/petroleum ether to give the title compound, m.p. 124.5 `.

EXAMPLE 2

This Example illustrates the preparation of :

1-(1,2,4-Triazol-1-yl)-2-phenyl-3-p-fluorophenyl-propan-2-ol (Compound No 3 of Table I)

p-Fluorobenzyl chloride (0.1 mol) in dry diethyl ether (100 ml) was added dropwise to magnesium turnings (0.11 g atoms) and the solution stirred vigorously until refluxing occurred. When all the magnesium had reacted, the solution was refluxed for a further 1 hour and then cooled to room temperature. Phenacyl

chloride (0.05 mol) in dry diethyl ether (50 ml) was added dropwise to the solution over 1 hour at such a rate as to maintain gentle reflux. The mixture was refluxed for 2 hours, cooled to room temperature and the mixture poured into ice/ammonium chloride solution to decompose the complex. The ethereal solution was washed several times with water (2 x 200 ml), dried ($Na_2SO_4$), and the solvent removed in vacuo to give, as a colourless oil, the crude chlorohydrin. The latter was dissolved in dimethyl-formamide (40 ml) and a solution of sodium triazole [prepared from sodium (0.05 g atoms) in methanol (20 ml) and 1,2,4-triazole (0.05 mol)] added dropwise at room temperature. After stirring at room temperature for 2 hours, the solution was warmed at 50 ` for 3 hours. The solvent was removed in vacuo and the mixture poured into water to give a cryystalline solid which was recrystallised from petroleum ether/chloroform to give the title compound, m.p. 116-8 `.

EXAMPLE 3

This Example illustrates the preparation of :

1,1-Diphenyl-2-(1,2,4-triazol-1-yl)-ethan-1-ol (Compound No 17 of Table I)

Stage 1. Bromobenzene (0.2 mol, 31.4 g) in sodium dry diethyl ether (200 ml) was added dropwise to magnesium (0.22 gram atoms, 5.3 g). After all the magnesium had reacted, phenacyl chloride (0.1 mol, 15.5 g) in diethyl ether (100 ml) was added dropwise and the solution stirred at room temperature for 1 hour. The reaction mixture was poured into saturated ammonium chloride solution, washed with water (3 x 150 ml), and dried ($Na_2SO_4$). Removal of the ether gave a pale yellow oil which solidified on standing. Recrystallisation from petroleum ether (60-80 `) gave 1,1-diphenyl-2-chloro-ethan-1-ol (60%) as a white crystalline solid, m.p. 56-57 `.

Stage 2. 1,2,4-Triazole (0.03 mol, 2.07 g) was added portionwise to a suspension of sodium hydride (0.03 mol, 0.72 g) in DMF (30 ml) and the solution stirred until effervescence ceased. 1,2-Diphenyl-2-chloro-ethan-1-ol (0.015 mol, 2.94g) in dimethylformamide (DMF; 10 ml) was added dropwise and the solution warmed at 100 ` for six hours. The reaction mixture was poured into water and a white solid crystallised out. This was filtered off, washed with water, dried, and recrystallised from ethanol to give the title compound as a white crystalline solid, m.p. 128-129 `.

EXAMPLE 4

This Example illustrates the preparation of :

2-Methyl-4-phenyl-5-(1,2,4-triazol-1-yl)-pentan-4-ol (Compound No 31 of Table I)

Stage 1. The Grignard reagent generated from isobutyl bromide (0.1 mol, 13.7 g) in sodium dry diethyl ether (50 ml) and magnesium turnings (0.11 g atoms; 2.6 g) was added dropwise to a solution of phenacyl chloride (0.05 mol, 7.7 g) in sodium dry diethyl ether (100 ml) so that gentle reflux was maintained. The solution was then stirred at room temperature for 1 hour and the magnesium complex destroyed by pouring into a saturated ammonium chloride solution (200 ml). The ethereal extract was washed with water (3 x 150 ml) and dried ($Na_2SO_4$). Removal of the solvent gave a colourless liquid which distilled at reduced pressure to give 2-methyl-4-phenyl-5-chloro-pentan-4-ol (70%), b.p. 86-88 `/0.01 mm Hg.

Stage 2. 1,2,4-Triazole (0.03 mol, 2.07 g) was added portionwise to 100% sodium hydride (0.03 mol, 0.72 g) in dry DMF (30 ml) and stirred at room temperature until the effervescence ceased. 2-Methyl-4-phenyl-5-chloro-pentan-4-ol (0.01 mol, 2.1 g) in dry DMF (10 ml) was added dropwise at room temperature and then the solution was stirred at 100 ` for 6 hours. On cooling to room temperature the solution was poured into water to precipitate out a solid which was recrystallised from petroleum (60-80 `)/chloroform giving the title compound (60%) as a white crystalline solid, m.p. 94-95 `.

EXAMPLE 5

This Example illustrates the preparation of :

1-(1,2,4-Triazol-1-yl)-2-o-chlorophenyl-2-p-fluorophenyl-ethan-2-ol (Compound No 45 of Table I)

A solution of dimethyl oxosulphonium methylide was prepared under nitrogen from sodium hydride (0.03

mol) and powdered trimethyl oxosulphonium iodide (0.03 mol) in dry dimethylsulphoxide (DMSO; 30 ml). A solution of o-chlorophenyl p-fluorophenyl ketone (0.025 mol) in DMSO (10 ml) was added dropwise at room temperature. The solution was then heated at 50 ` for 1.5 hours, cooled to room temperature and poured into water. The solution was extracted with diethyl ether (100 ml), washed with water (3 x 100 ml), and dried over anhydrous sodium sulphate. Removal of the solvent gave 1-o-chlorophenyl-1-p-fluorophenyl ethylene oxide (90%) as a colourless liquid.

1,2,4-Triazole (0.04 mol) was added portionwise to sodium hydride (0.04 mol) in DMF (40 ml) and the solution stirred at room temperature until effervescence ceased. 1-o-Chlorophenyl-1-p-fluorophenyl ethylene oxide (0.02 mol) in DMF (10 ml) was addred dropwise and the solution stirred at 80 ` for 4 hours. The solution was poured into water and triturated with petroleum ether to give a white crystalline solid which was filtered off and dried. Recrystallisation from petroleum ether (60-80 `)/methylene chloride gave the title compound (70%) as a white crystalline solid, m.p. 115-116 `.

EXAMPLE 6

This Example illustrates the preparation of :

2,2-Dimethyl-3-(2-methoxybenzyl)-4-(1,2,4-triazol-1-yl)-butan-3-ol (Compound No 130 of Table I)

Stage 1. Potassium t-butoxide (19 g) was dissolved in dimethylsulphoxide (200 ml; dried by distilling from calcium hydride and sodamide) and pinacolone (15 g; freshly distilled from calcium hydride) was added under argon to give a yellow solution. o-Methoxyphenyl iodide (10 g) was then added and a brown colour rapidly developed. The solution was stirred for 1.5 hours before pouring into water (1 litre), acidifying the mixture with 2M hydrochloric acid and extracting it with diethyl ether. Evaporation of the dried ($MgSO_4$) ethereal solution gave a yellow liquid (11.8 g) b.p. 88-93 `C/0.9 mm. The distillate solidified to give 2,2-dimethyl- 4-(o-methoxyphenyl)butan-3-one (6 g).

Stage 2. Thioanisole (3.3 g) was added to diazobicyclo-octane (3.5 g) in dry tetrahydrofuran (THF) under argon and the colourless solution was cooled in an ice/salt bath. 1.6M-butyl lithium solution (20 ml) in hexane was then added over 10 minutes at 0 to 2 `C. After stirring the yellow solution for 15 minutes, a solid was precipitated. The mixture was stirred for a further 45 minutes in the ice bath and then it was allowed to warm up to room temperature. The mixture was then cooled in the ice bath and a solution of the product (5 g) of stage 1 in dry THF (25 ml) was added at 0 to 5 `C. When the addition was complete, the resultant yellow solution was allowed to stand overnight, poured into water, acidified with 2M-hydrochloric acid and extracted with diethyl ether. The ethereal solution was washed well with water, dried ($MgSO_4$) and evaporated to give a yellow liquid (8.8 g) which solidified on standing. Recrystallisation from petroleum ether (60-80 `) gave 2,2-dimethyl-3-hydroxy-3-(o-methoxybenzyl)-4-thiophenylbutane (3.1 g), m.p. 74-75 `C.

Stage 3. The product (2.5 g) of Stage 2 was added to a stirred suspension of trimethyl oxonium tetrafluoroborate (1.3 g) in methylene chloride (25 ml). After about 1 hour a clear solution was obtained. The solvent was then removed on the rotavaporator to give a pale organe gum, which was dissolved in dry DMF (10 ml) and the solution added to a solution of 1,2,4-triazole sodium salt (1.2 g) in DMF (15 ml). [The solution was prepared by washing sodium hydride with dry diethyl ether, suspending it in dry DMF and adding the triazole]. The reaction mixture was then stirred at 120 `C for 2.5 hours. The reaction mixture was then quenched by pouring into water (100 ml) and the emulsion was extracted with diethyl ether (3 x 50 ml). The ethereal solution was washed well with water, dried ($MgSO_4$) and evaporated to give a pale yellow liquid. The mixture was subjected to dry column chromatography on silica eluting with diethyl ether to give a colourless liquid which solidified on trituration with diethyl ether. Recrystallation from petroleum ether (60-80 `) gave the title compound (0.5g; 23%), m.p. 113-116 `.

EXAMPLE 7

This Example illustrates the preparation of :

1-(1,2,4-Triazol-1-yl)-2-(2-chloro-4-methoxy-phenyl)-2-(4-fluoro-phenyl)-ethan-2-ol (Compound No 294 of Table I)

Stage 1. 3-chloroanisole (0.1 mol) was added dropwise to a suspension of aluminium chloride (0.11 mol) in carbon disulphide (40 ml) at room temperature and stirred for 30 minutes until it had dissolved. The mixture was cooled in ice and 4-fluorobenzoyl chloride (0.1 mol) added dropwise. After complete addition the

solution was stirred for 1 hour at room temperature and refluxed for 1 hour. The solution was poured into ice (200 ml) containing CHCl (20 ml), extracted with ether (200 ml), washed with water (3 x 150 ml), and dried over anhydrous sodium sulphonate. Removal of the solvent gave an oil which was purified by column chromatography silica K6O eluted with toluene/petrol 1:1 to give (2-chloro-4-methoxy-phenyl)-4-fluorophenyl-ketone (40%) as colourless crystalline solid m.p. 31 `.

Stage 2. A solution of dimethyl sulphonium methylide was prepared under nitrogen at 0 `C from sodium hydride (0.03 mol using 80% suspension in oil), trimethyl sulphonium iodide (0.03 mol) in dry dimethyl sulphoxide (30 ml) and dry tetrahydrofuran (30 ml). A solution of (2-chloro-4-methoxyphenyl)-4-fluorophenyl ketone (0.02 mol) in tetrahydrofuran (20 ml) was added at 0 `, stirred for 30 minutes at 0 `, and allowed to warm-up to toom temperature. The solution was poured into water, extracted with diethyl ether (150 ml), washed with water (3 x 100 ml) and dried over anhydrous mangnesium sulphate. Removal of the solvent gave 1-(2-chloro-4-methoxy-phenyl)-1-(4-fluorophenyl)-ethylene oxide (90%) as a colourless oil.

Stage 3. 1,2,4-Triazole (0.03 mol) was added portionwise to sodium hydride (0.03 mol) in dimethyl formamide (30 ml) and the solution stirred at room temperature until the effervescence ceased. 1-(2-chloro-4-methoxyl-phenyl)-1-(4-fluorophenyl)-ethylene oxide (0.015 mol) in dimethyl formamide (10 ml) was added dropwise and the solution stirred at 80 ` for 4 hours. The solution was poured into water and triturated with petroleum ether to an off-white solid which was filtered off and dried. Recrystallisation from petroleum ether/chloroform gave the title compound (65%) as a white crystalline solid m.p. 138-140 `.

EXAMPLE 8

This Example illustrates the preparation of :

1-(1,2,4-Triazol-1-yl)-2-(4-chlorophenyl)-3,3-dimethyl-butan-2-ol (Compound No 196 of Table I)

Stage 1. Trimethyl acetonitrile (0.1 mol) in dry tetrahydrofuran (50 ml) was added dropwise with stirring to the Grignard reagent prepared from 4-chloro-iodobenzene (0.11 mol) and magnesium turnings(0.11 g atoms) in sodium dry ether (50 ml) at such a rate as to retain gentle reflux. The solution was refluxed for 4 hours, cooled to room temperature, and water (40 ml) added carefully. 2N $H_2SO_4$ (50 ml) was added and the ether solution washed with water (3 x 100 ml) and dried over anhydrous magnesium sulphate. Removal of the solvent gave a yellow oil which was distilled at the water pump to give tertiary butyl 4-chlorophenyl ketone (40%) as a colourless liquid b.p. 116-128 `/1995 Pa (15 mm Hg).

Stage 2. A solution of dimethyl oxosulphonium methylide was prepared under nitrogen from sodium hydride (0.03 mol) and powdered trimethyl oxosulphonium iodide (0.03 mol) in dry dimethyl sulphoxide (40 ml). A solution of 4-chlorophenyl tertiary butyl ketone (0.025 mol) in dry dimethyl sulphoxide (10 ml) was added dropwise at room temperature and the solution heated at 50 `C for 2.5 hours. After cooling to room temperature the solution was extracted with ether (150 ml), washed with water and dried over anhydrous sodium sulphate. Removal of the solvent gave 1-(4-chlorophenyl)-1-t-butyl-ethylene oxide (95%) as a colourless liquid.

Stage 3. 1,2,4-Triazole (0.04 mol) was added portionwise to sodium hydride (0.04 mol) in dimethyl formamide (40 ml) and the solution stirred at room temperature until the effervescence ceased. 1-(4-chlorophenyl)-1-t-butyl-ethylene oxide (0.02 mol) in dimethyl formamide (10 ml) was added dropwise and the solution stirred at 60 ` for 3 hours. The solution was poured into water and triturated with petroleum ether to give a white solid which recrystallised from 60/80 petroleum ether gave the title compound (65%) m.p. 70-3 `.

EXAMPLE 9

This Example illustrates the preparation of :

1-(1,2,4-Triazol-1-yl)-bis-2-(4-fluorophenyl)-2-methoxy-ethane

Stage 1. A solution of dimethyl oxosulphonium methylide was prepared under nitrogen from sodium hydride 0.1 mol) and powdered trimethyl oxosulphonium iodide (0.1 mol) in dry dimethyl sulphoxide (100 ml). A solution of 4,4'-difluorobenzophenone (0.08 mol) in dimethyl sulphoxide (40 ml) was added dropwise at room temperature and the solution heated at 50 ` for 2 hours. After cooling to room temperature the solution was extracted with diethyl ether (400 ml), washed with water (3 x 250 ml), and dried over anhydrous sodium sulphate. Removal of the solvent gave 1,1-bis-(4-fluorophenyl)-ethylene oxide (95%) as a colourless oil.

Stage 2. 1,2,4-Triazole (0.1 mol) was added portionwise to sodium hydride (0.1 mol) in dimethyl formamide (100 ml) and the solution stirred at room temperature until the effervescence ceased. 1,1-bis-(4-fluorophenyl)-ethylene oxide (0.05 mol) in dimethyl formamide (25 ml) was added dropwise and the solution stirred at 80 ` for 4 hours. The solution was poured into water and triturated with petroleum ether to give a white crystalline solid which was filtered off and dried. Recrystallisation from petroleum ether (60-80 `)/chloroform gave 1-(1,2,4-triazol-1-yl)-bis-2-(4-fluorophenyl)-ethan-2-ol (70%) as a white crystalline solid m.p. 170-1 `.

Stage 3. Sodium hydride (0.02 mol) was suspended in dry tetrahydrofuran (50 ml) and 1-(1,2,4-triazol-1-yl)-bis-2-(4-fluorophenyl)-ethan-2-ol (0.02 mol) added portionwise at room temperature. The solution was warmed to 50 ` to complete the reaction. After cooling to room temperature methyl iodide (0.04 mol) was added dropwise at 20 ` and the reaction stirred at room temperature for 24 hours. The reaction was poured into water (100 ml), extracted with diethyl ether (150 ml), washed with water (2 x 100 ml), and dried over anhydrous sodium sulphate. Removal of the solvent gave an oil which solidified on standing. Recrystallisation from petroleum-ether/chloroform gave the title compound (90%) as a white crystalline solid. m.p. 100 `.

EXAMPLE 10

An emulsifiable concentrate was made up by mixing the ingredients, and stirring the mixture until all the constituents were dissolved.

| | |
|---|---|
| Compound of Example 1 | 10% |
| Ethylene dichloride | 40% |
| Calcium dodecylbenzenesulphate | 5% |
| "Lubrol" L | 10% |
| "Aromasol" H | 35% |

EXAMPLE 11

A composition in the form of grains readily dispersible in a liquid, e.g. water, was prepared by grinding together the first three ingredients in the presence of added water and then mixing in the sodium acetate. The resultant mixture was dried and passed through a British Standard mesh sieve, size 44-100, to obtain the desired size of grains.

| | |
|---|---|
| Compound of Example 2 | 50% |
| "Dispersol" T | 25% |
| "Lubrol" APN5 | 1.5% |
| Sodium acetate | 23.5% |

EXAMPLE 12

The ingredients were all ground together to produce a powder formulation readily dispersible in liquids.

| | |
|---|---|
| Compound of Example 3 | 45% |
| "Dispersol" T | 5% |
| "Lissapol" NX | 0.5% |
| "Cellofas" B600 | 2% |
| Sodium acetate | 47.5% |

EXAMPLE 13

The active ingredient was dissolved in a solvent and the resultant liquid was sprayed on to the granules

of China clay. The solvent was then allowed to evaporate to produce a granular composition.

| | |
|---|---|
| Compound of Example 4 | 5% |
| China clay granules | 95% |

EXAMPLE 14

A composition suitable for use as a seed dressing was prepared by mixing the three ingredients.

| | |
|---|---|
| Compound of Example 1 | 50% |
| Mineral oil | 2% |
| China clay | 48% |

EXAMPLE 15

A dusting powder was prepared by mixing the active ingredient with talc.

| | |
|---|---|
| Compound of Example 2 | 5% |
| Talc | 95% |

EXAMPLE 16

A Col formulation was prepared by ball-milling the constituents set out below and then forming an aqueous suspension of the ground mixture with water.

| | |
|---|---|
| Compound of Example 3 | 40% |
| "Dispersol" T | 10% |
| "Lubrol" APN5 | 1% |
| Water | |

EXAMPLE 17

A dispersible powder formulation was made by mixing together the ingredients set out below and then grinding the mixture until all were thoroughly mixed.

| | |
|---|---|
| Compound of Example 4 | 25% |
| "Aerosol" OT/B | 2% |
| "Dispersol" A.C. | 5% |
| China clay | 28% |
| Silica | 40% |

EXAMPLE 18

This Example illustrates the preparation of a dispersible powder formulation. The ingredients were mixed and the mixture then ground in a comminution mill.

| | |
|---|---|
| Compound of Example 1 | 25% |
| "Perminal" BX | 1% |
| "Dispersol" T | 5% |
| Polyvinylpyrrolidone | 10% |
| Silica | 25% |
| China clay | 34% |

EXAMPLE 19

The ingredients set out below were formulated into a dispersible powder by mixing then grinding the ingredients.

| | |
|---|---|
| Compound of Example 2 | 25% |
| "Aerosol" OT/B | 2% |
| "Dispersol" A | 5% |
| China clay | 68% |

In Examples 10 to 19 the proportions of the ingredients given are by weight.

There now follows an explanation of the compositions or substances represented by the various Trade Marks and Trade Names mentioned above.

| | |
|---|---|
| LUBROL L : | a condensate of nonyl phenol 1 mole) with ethylene oxide (13 moles) |
| AROMASOL H : | a solvent mixture of alkylbenzenes |
| DISPERSOL T & AC : | a mixture of sodium sulphate and a condensate of formaldehyde with sodium naphthalene sulphonate |
| LUBROL APN5 : | a condensate of nonyl phenol (1 mole) with naphthalene oxide (5.5 moles) |
| CELLOFAS B600 : | a sodium carboxymethyl cellulose thickener |
| LISSAPOL NX : | a condensate of nonyl phenol (1 mole) with ethylene oxide (8 moles) |
| AEROSOL OT/B : | dioctyl sodium sulphosuccinate |
| PERMINAL BX : | a sodium alkyl naphthalene sulphonate |

**Claims**

**1.** A method of combating fungi on non-agricultural materials which comprises applying to a material, or to the locus of a material a sufficient amount to produce a fungal-combating effect of a compound of general formula (I):

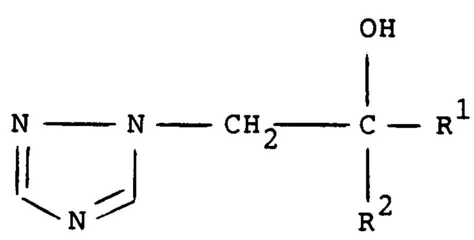

wherein $R^1$ is $C_{1-6}$ alkyl, or cycloalkyl having up to 6 carbon atoms or phenyl, and $R^2$ is phenyl or benzyl; the phenyl of $R^1$ and the phenyl or phenyl moiety of the benzyl of $R^2$ being optionally substituted with halogen, $C_{1-5}$ alkyl, $C_{1-4}$ alkoxy, halo-$C_{1-4}$ alkyl, halo-$C_{1-4}$ alkoxy, nitro, phenyl, phenoxy, benzyl, optionally halo-substituted benzyloxy, alkylenedioxy halo alkylenedioxy, amino, mono- or di- $C_{1-4}$ alkylamino, hydroxy, morpholino or carboxy or an alkyl ester thereof, and/or the alkyl moiety of the benzyl is optionally substituted with one $C_{1-2}$ alkyl; or an acid addition salt, ester or metal complex thereof as hereinbefore defined.

**2.** A method as claimed in claim I wherein $R^1$ is $C_{1-4}$ alkyl and $R^2$ is optionally substituted benzyl.

**3.** A method as claimed in claim 1 wherein $R^1$ is optionally substituted phenyl and $R^2$ is optionally substituted benzyl.

**4.** A method as claimed in claim 1 wherein each of $R^1$ and $R^2$, which may be the same or different, is optionally substituted phenyl.

**5.** A method as claimed in claim 1, 2 or 5 wherein $R^1$ is $C_{1-4}$ alkyl and $R^2$ is optionally substituted phenyl.

**6.** A method as claimed in claim 1 wherein $R^1$ is butyl.

**7.** A method as claimed in claim 6 wherein $R^1$ is t-butyl.

**8.** A method according to claim 4 wherein $R^1$ and $R^2$ are both halophenyl.

**9.** A method according to claim 8 wherein $R^1$ and $R^2$, which may be the same or different, are each chlorophenyl or fluorophenyl.

**10.** A method according to claim 8 or 9 wherein at least one phenyl ring also bears one or more alkoxy groups.

**11.** A method according to claim 5 wherein $R^1$ is alkyl and $R^2$ is halophenyl.

**12.** A method according to claim 11 wherein $R^1$ is n-butyl or t-butyl and $R^2$ is p-chlorophenyl.

**13.** A method according to claim 10 wherein $R^1$ is 2-chloro-4-methoxyphenyl and $R^2$ is 2-chloro-, 2-fluoro, 4-chloro- or 4-fluoro-phenyl.

**14.** A method as claimed in claim 1 wherein the compound is:

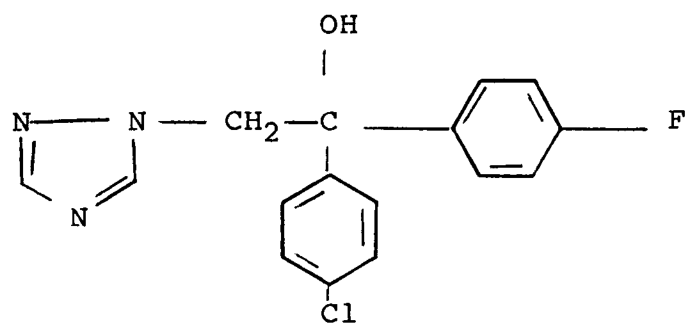

**15.** A method as claimed in claim 1 wherein the compound has the formula:

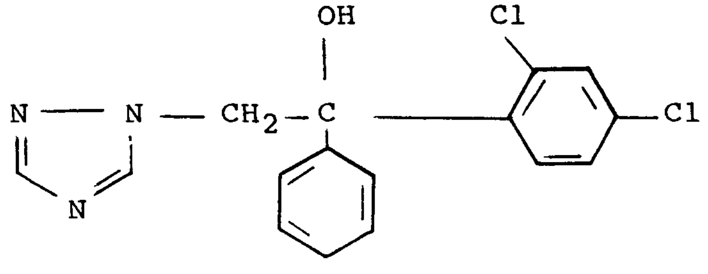

**16.** A method as claimed in claim 1 wherein the compound has the formula:

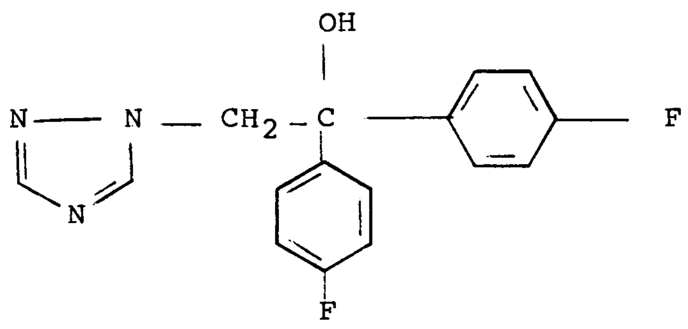

**17.** A method as claimed in claim 1 wherein the compound has the formula:

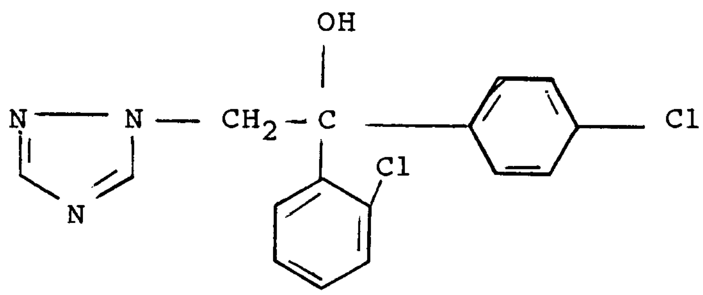

**18.** A method as claimed in claim 1 wherein the compound has the formula:

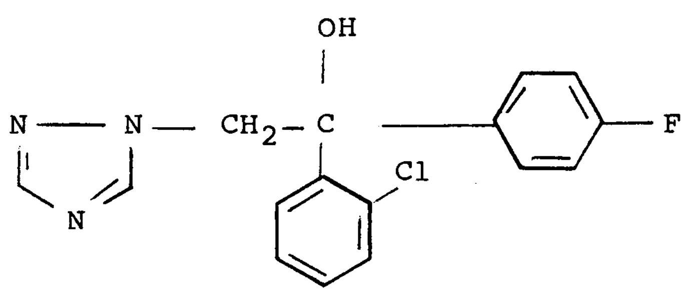

**19.** A method as claimed in claim 1 wherein the compound has the formula:

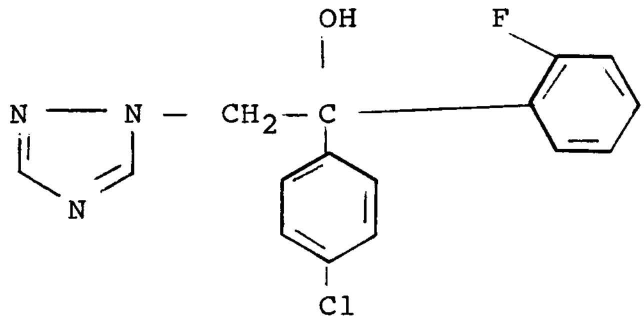

**20.** A method as claimed in claim 1 wherein the compound has the formula:

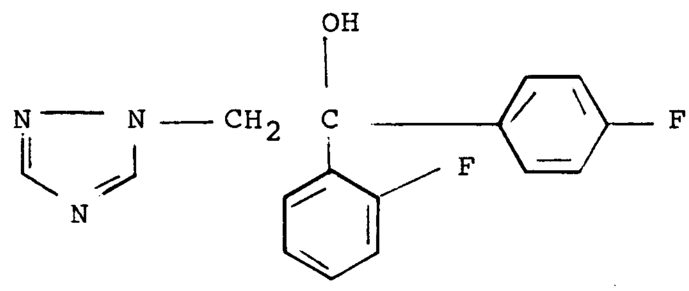

**21.** A method as claimed in claim 1 wherein the compound has the formula:

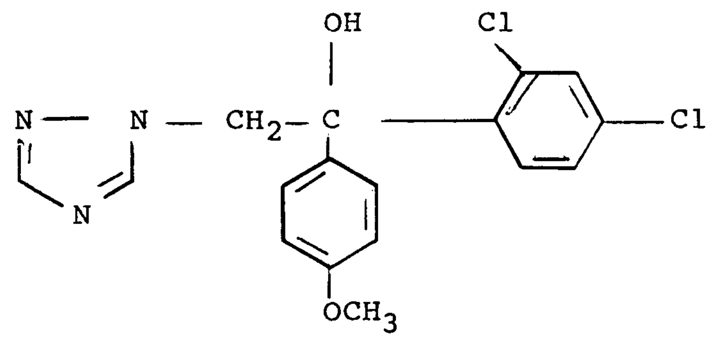

**22.** A method as claimed in claim 1 wherein the compound has the formula:

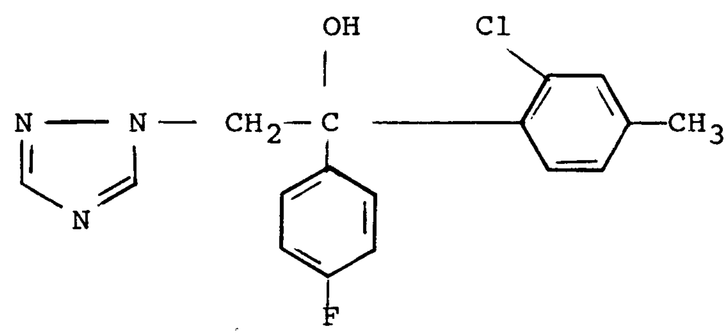

**23.** A method as claimed in claim 1 wherein the compound has the formula:

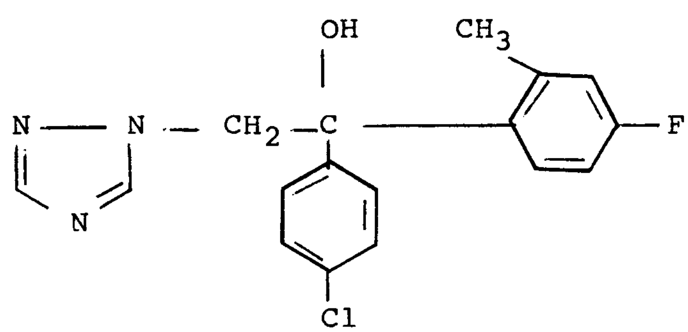

**24.** A method as claimed in claim 1 wherein the compound has the formula:

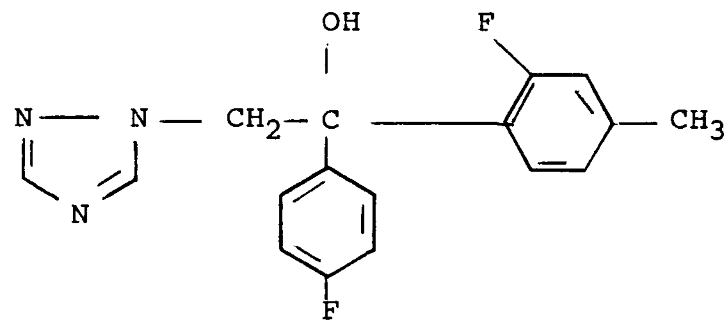

**25.** A method as claimed in claim 1 wherein the compound has the formula:

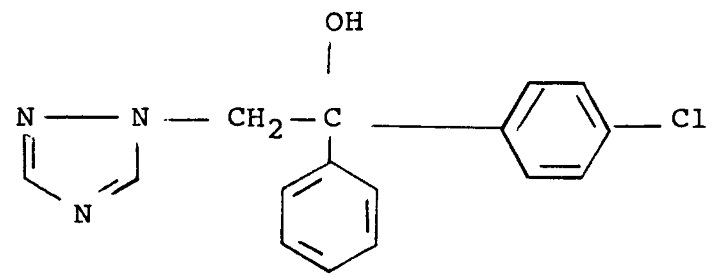

**26.** A method as claimed in claim 1 wherein the compound has the formula:

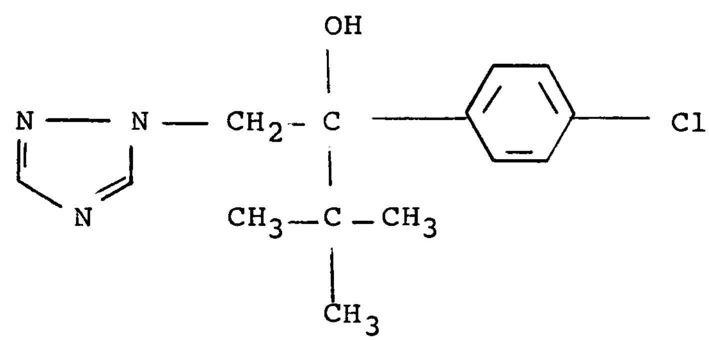

**27.** A method as claimed in claim 1 wherein the compound has the formula:

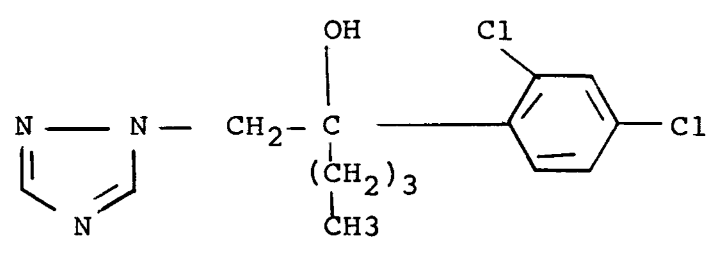

**28.** A method as claimed in claim 1 wherein the compound has the formula:

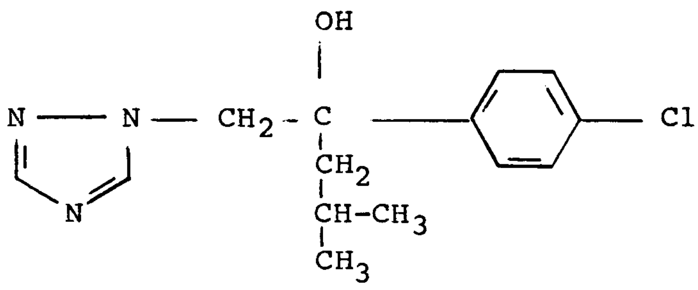

**29.** A method as claimed in claim 1 wherein the compound has the formula:

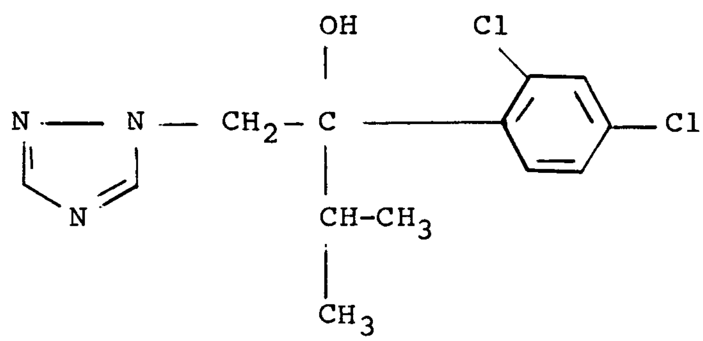

**30.** A method as claimed in claim 1 wherein the compound has the formula:

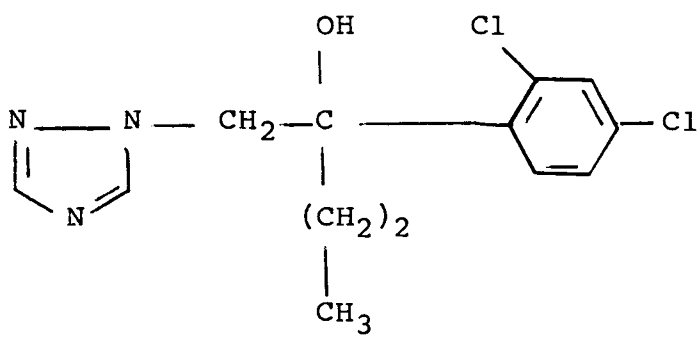

**31.** A method as claimed in claim 1 wherein the compound has the formula:

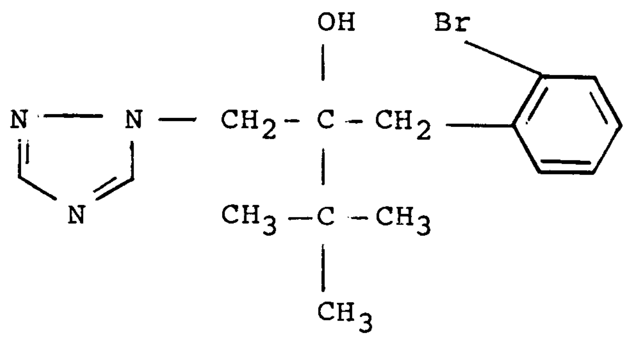

**32.** A method as claimed in claim 1 wherein the compound has the formula:

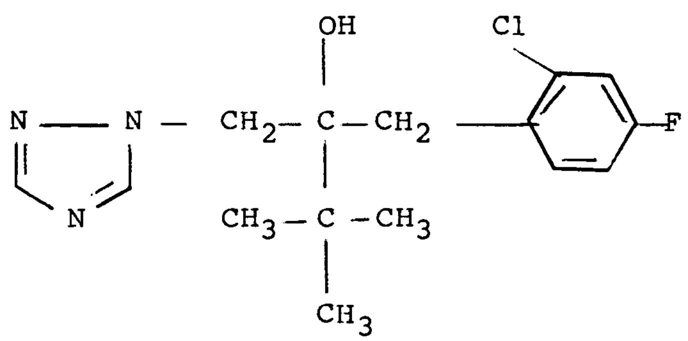

**33.** A method as claimed in claim 1 wherein the compound has the formula:

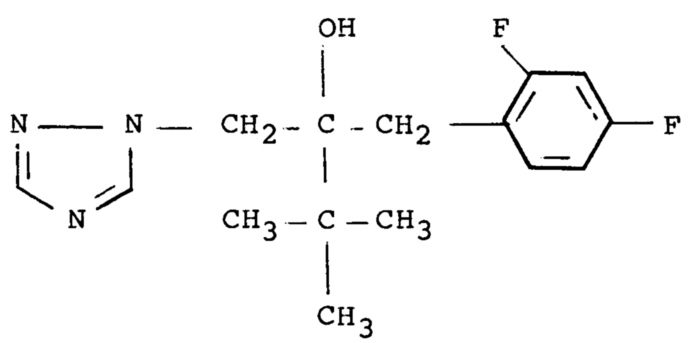

**34.** A method as claimed in claim 1 wherein the compound has the formula:

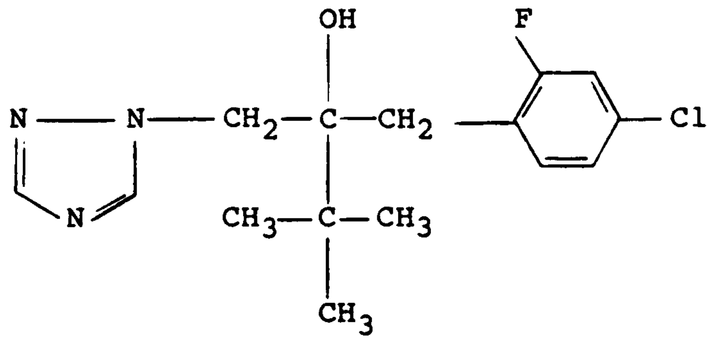

35. A method as claimed in claim 1 wherein the compound has the formula:

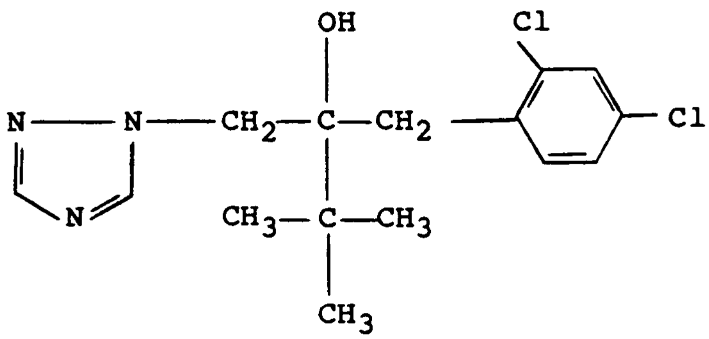

36. A method as claimed in claim 1 wherein the compound has the formula:

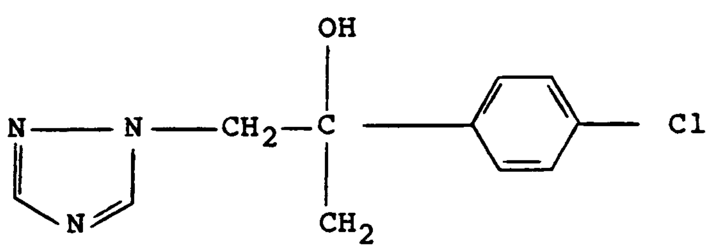

37. A method as claimed in claim 1 wherein the compound has the formula:

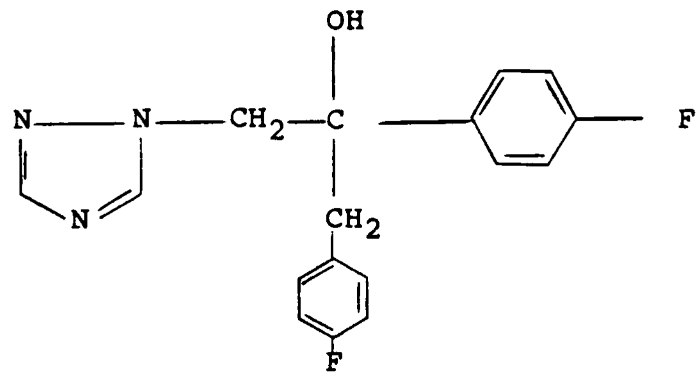

**38.** A method as claimed in claim 1 wherein the compound has the formula:

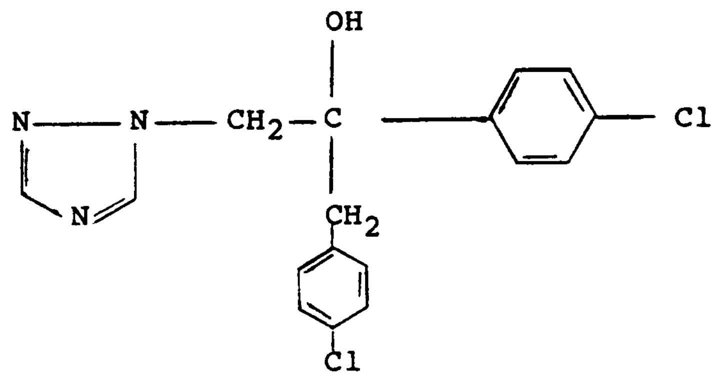

**39.** A method as claimed in claim 1 wherein the compound has the formula:

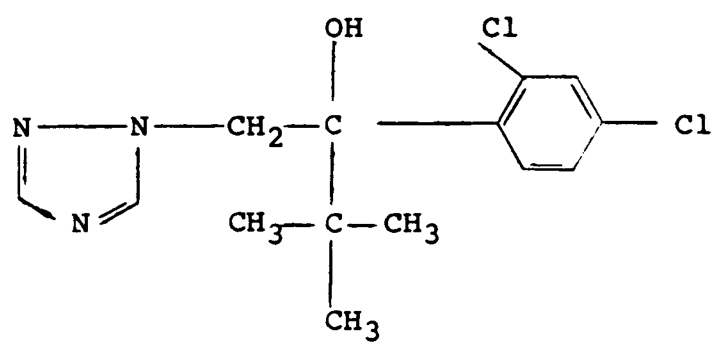

**40.** A method as claimed in claim 1 wherein the compound has the formula:

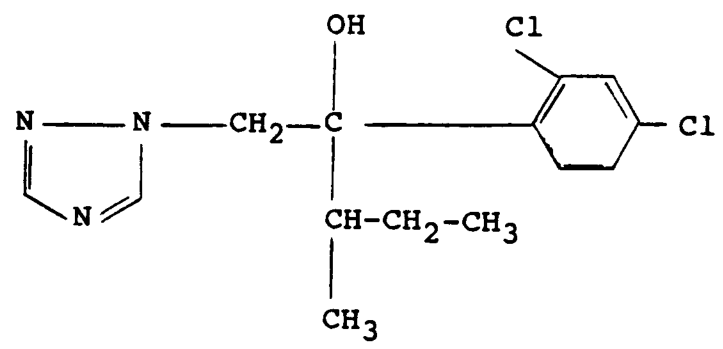

**41.** A method as claimed in claim 1 wherein the compound has the formula:

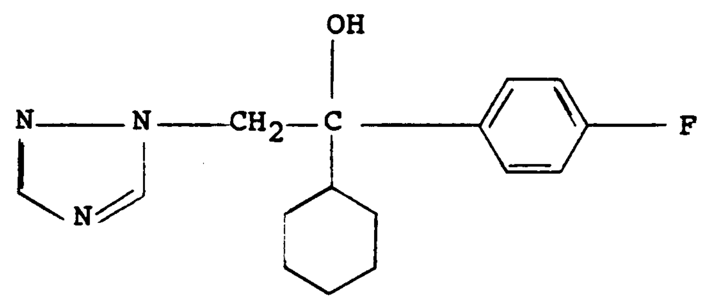

**42.** A method as claimed in claim 1 wherein the compound has the formula:

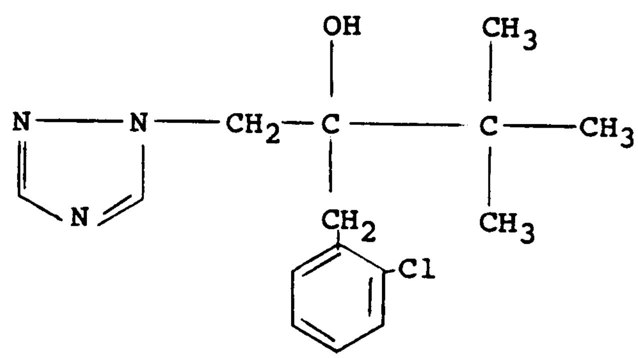

**43.** A method as claimed in claim 1 wherein the compound has the formula:

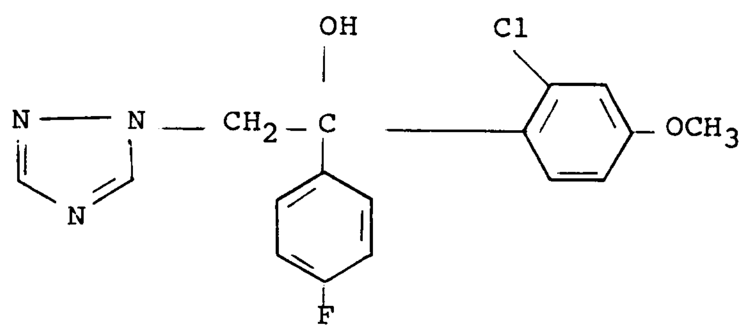

44. Wood, hides, leather and paint films when treated by the method of claim 27

45. Wood, hides, leather and paint films treated with a compound as defined in claim 1 or an acid addition salt, ester or metal complex thereof.

## Revendications

1. Procédé pour combattre des champignons sur des matériaux non agricoles, qui consiste à appliquer à un matériau, ou sur le lieu d'un matériau, une quantité suffisante pour produire un effet antifongique d'un composé de formule générale (I) :

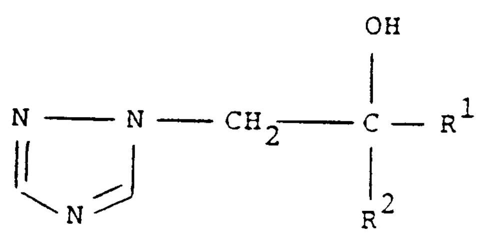

dans laquelle $R^1$ est un groupe alkyle en $C_1$ à $C_6$ ou un groupe cycloalkyle ayant jusqu'à 6 atomes de carbone ou un groupe phényle, et $R^2$ est un groupe phényle ou benzyle ; le groupe phényle de $R^1$ et le groupe phényle ou la portion phényle du groupe benzyle de $R^2$ étant facultativement substitués avec un halogène, un radical alkyle en $C_1$ à $C_5$, alkoxy en $C_1$ à $C_4$, halogénalkyle en $C_1$ à $C_4$, halogénalkoxy en $C_1$ à $C_4$, nitro, phényle, phénoxy, benzyle, benzyloxy facultativement halogéné, alkylènedioxy, alkylènedioxy halogéné, amino, mono- ou di-(alkyle en $C_1$ à $C_4$)amino, hydroxy, morpholino ou carboxy ou son ester d'alkyle, et/ou la portion alkyle du groupe benzyle est facultativement substituée avec un radical alkyle en $C_1$ ou $C_2$ ; ou un sel d'addition d'acide, un ester ou un complexe métallique de ce composé tel que défini précédemment.

2. Procédé suivant la revendication 1, dans lequel $R^1$ est un groupe alkyle en $C_1$ à $C_4$ et $R^2$ est un groupe benzyle facultativement substitué.

3. Procédé suivant la revendication 1, dans lequel $R^1$ est un groupe phényle facultativement substitué et $R^2$ est un groupe benzyle facultativement substitué.

4. Procédé suivant la revendication 1, dans lequel chacun de $R^1$ et $R^4$, qui peuvent être identiques ou différents, est un groupe phényle facultativement substitué.

5. Procédé suivant la revendication 1, 2 ou 5, dans lequel $R^1$ est un groupe alkyle en $C_1$ à $C_4$ et $R^2$ est un groupe phényle facultativement substitué.

6. Procédé suivant la revendication 1, dans lequel $R^1$ est le groupe butyle.

7. Procédé suivant la revendication 6, dans lequel $R^1$ est le groupe tertio-butyle.

8. Procédé suivant la revendication 4, dans lequel $R^1$ et $R^2$ sont tous deux des groupes halogénophényle.

9. Procédé suivant la revendication 8, dans lequel $R^1$ et $R^2$, qui peuvent être identiques ou différents, représentent chacun un groupe chlorophényle ou fluorophényle.

10. Procédé suivant la revendication 8 ou 9, dans lequel au moins un noyau phényle porte aussi un ou plusieurs groupes alkoxy.

11. Procédé suivant la revendication 5, dans lequel $R^1$ est un groupe alkyle et $R^2$ est un groupe halogénophényle.

**12.** Procédé suivant la revendication 11, dans lequel $R^1$ est un groupe n-butyle ou tertio-butyle et $R^2$ est un groupe p-chlorophényle.

**13.** Procédé suivant la revendication 10, dans lequel $R^1$ est le groupe 2-chloro-4-méthoxyphényle et $R^2$ est le groupe 2-chloro-, 2-fluoro-, 4-chloro- ou 4-fluorophényle.

**14.** Procédé suivant la revendication 1, dans lequel le composé est :

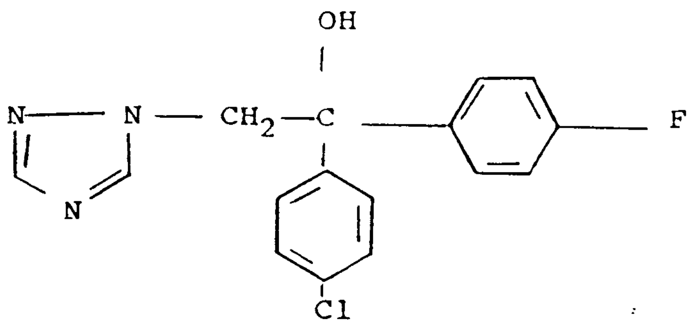

**15.** Procédé suivant la revendication 1, dans lequel le composé répond à la formule :

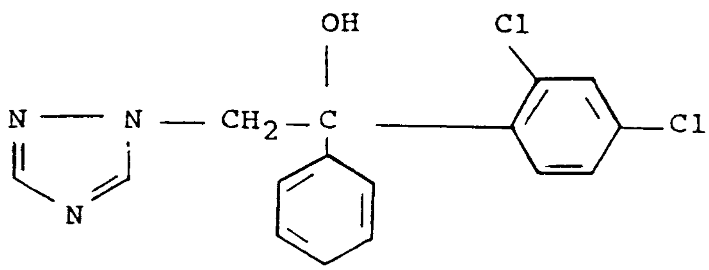

**16.** Procédé suivant la revendication 1, dans lequel le composé répond à la formule :

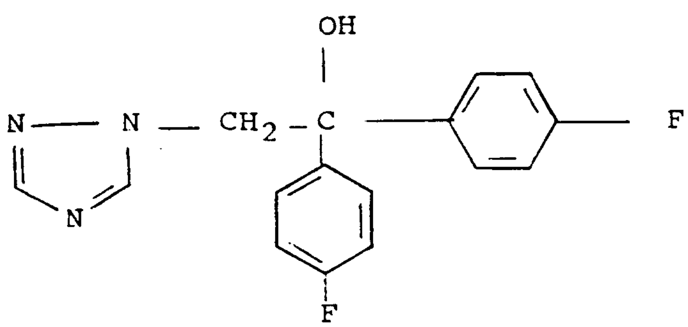

**17.** Procédé suivant la revendication 1, dans lequel le composé répond à la formule :

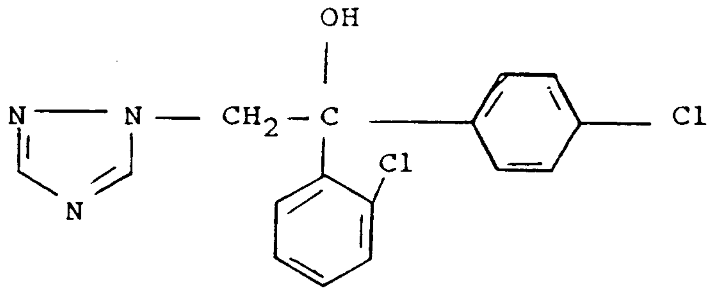

**18.** Procédé suivant la revendication 1, dans lequel le composé répond à la formule :

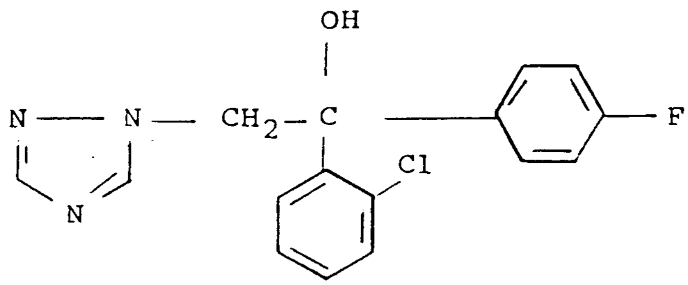

**19.** Procédé suivant la revendication 1, dans lequel le composé répond à la formule :

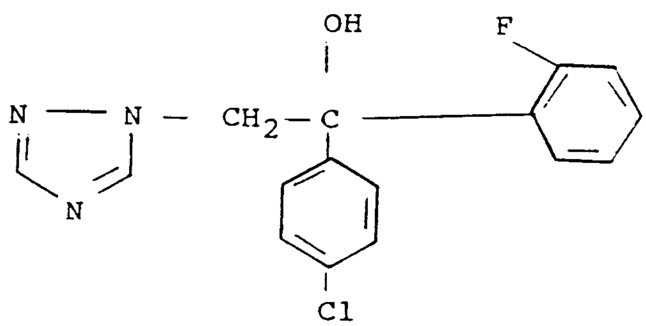

**20.** Procédé suivant la revendication 1, dans lequel le composé répond à la formule :

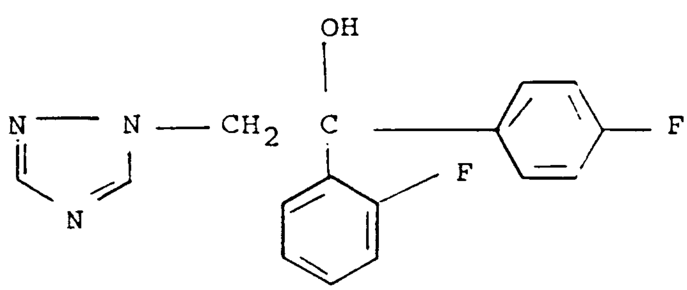

**21.** Procédé suivant la revendication 1, dans lequel le composé répond à la formule :

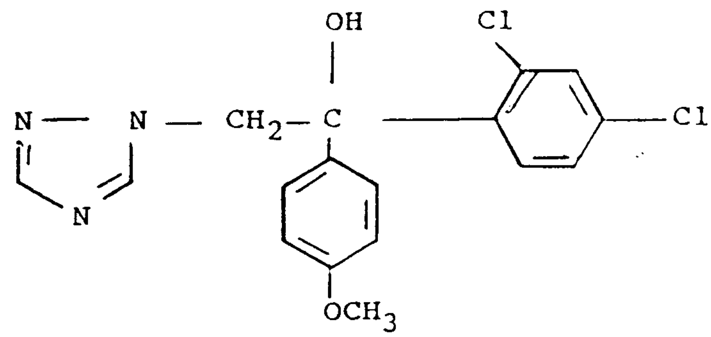

22. Procédé suivant la revendication 1, dans lequel le composé répond à la formule :

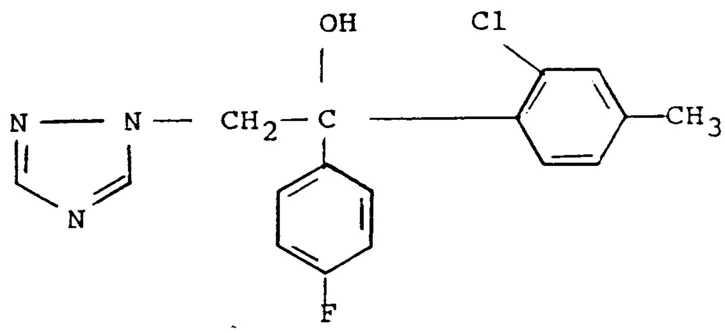

23. Procédé suivant la revendication 1, dans lequel le composé répond à la formule :

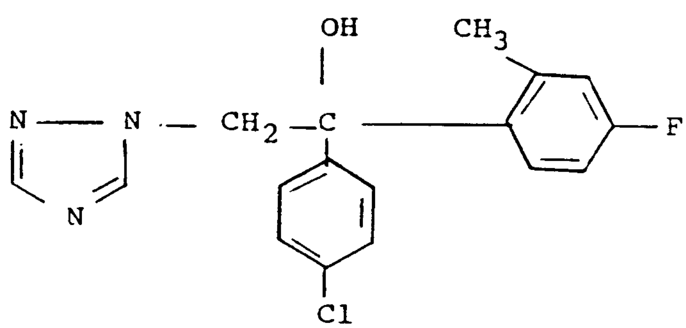

24. Procédé suivant la revendication 1, dans lequel le composé répond à la formule :

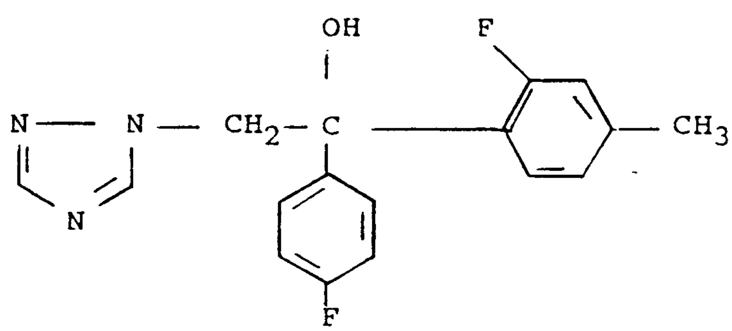

25. Procédé suivant la revendication 1, dans lequel le composé répond à la formule :

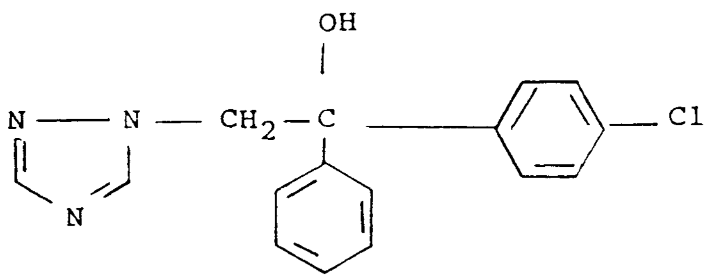

26. Procédé suivant la revendication 1, dans lequel le composé répond à la formule :

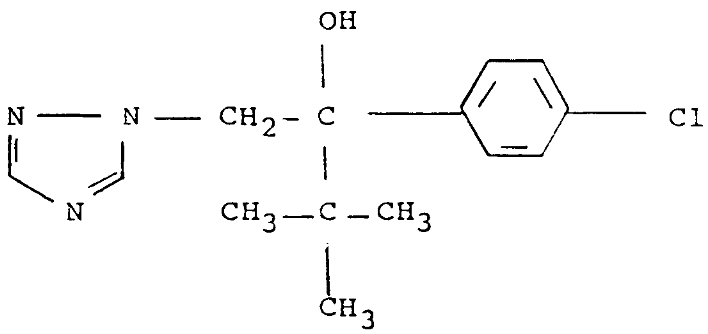

27. Procédé suivant la revendication 1, dans lequel le composé répond à la formule :

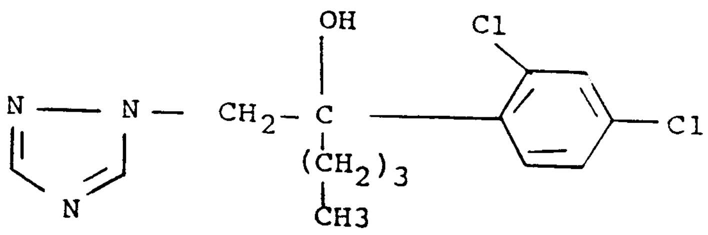

28. Procédé suivant la revendication 1, dans lequel le composé répond à la formule :

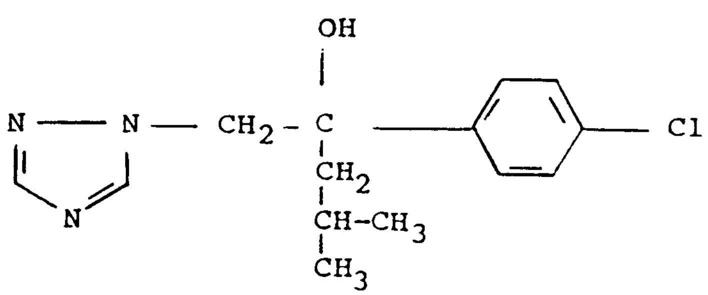

29. Procédé suivant la revendication 1, dans lequel le composé répond à la formule :

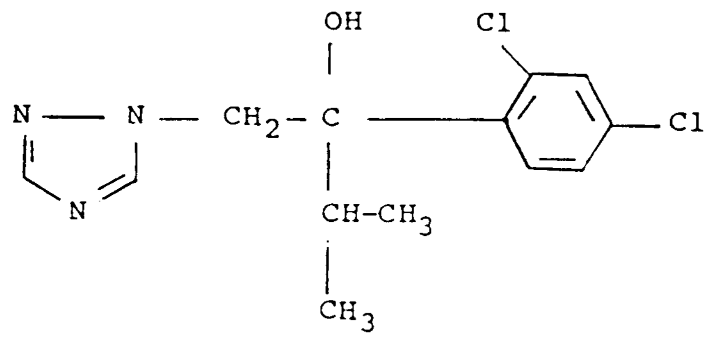

30. Procédé suivant la revendication 1, dans lequel le composé répond à la formule :

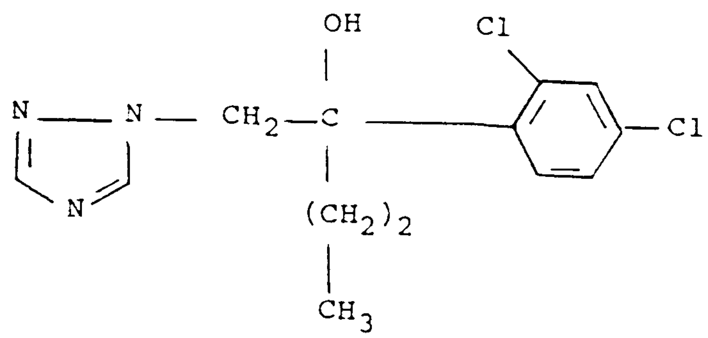

31. Procédé suivant la revendication 1, dans lequel le composé répond à la formule :

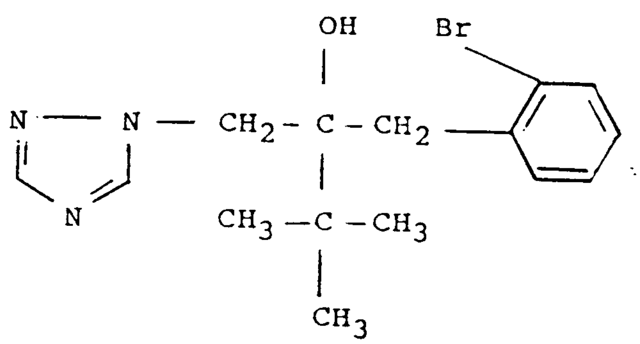

32. Procédé suivant la revendication 1, dans lequel le composé répond à la formule :

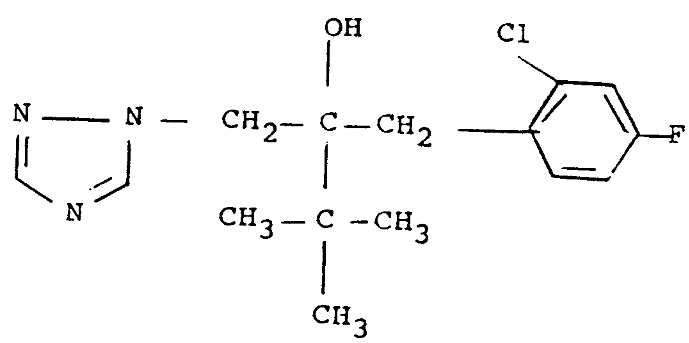

33. Procédé suivant la revendication 1, dans lequel le composé répond à la formule :

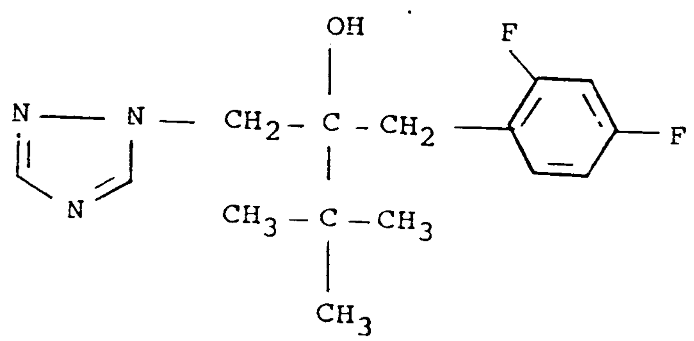

34. Procédé suivant la revendication 1, dans lequel le composé répond à la formule :

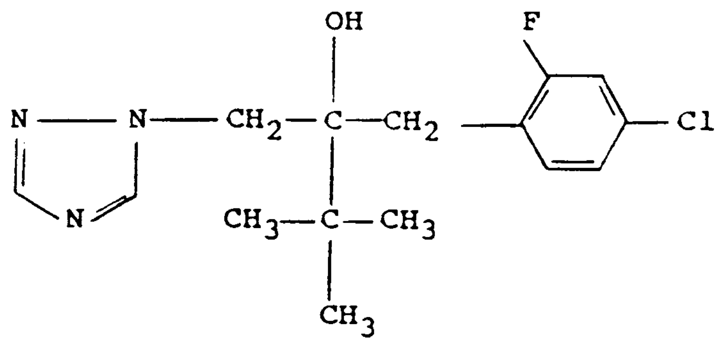

35. Procédé suivant la revendication 1, dans lequel le composé répond à la formule :

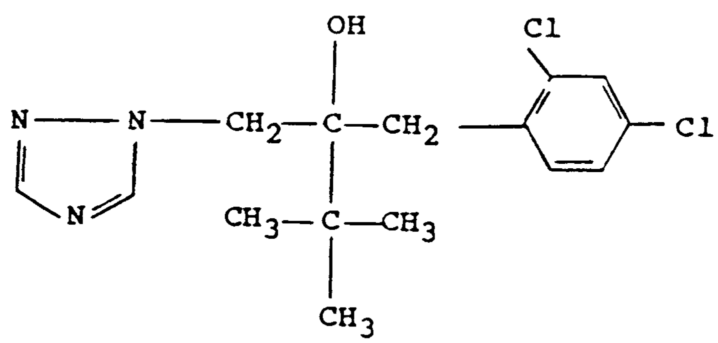

36. Procédé suivant la revendication 1, dans lequel le composé répond à la formule :

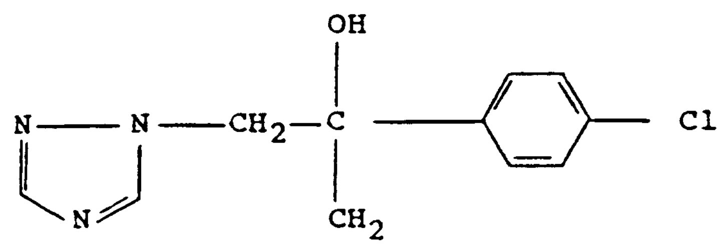

**37.** Procédé suivant la revendication 1, dans lequel le composé répond à la formule :

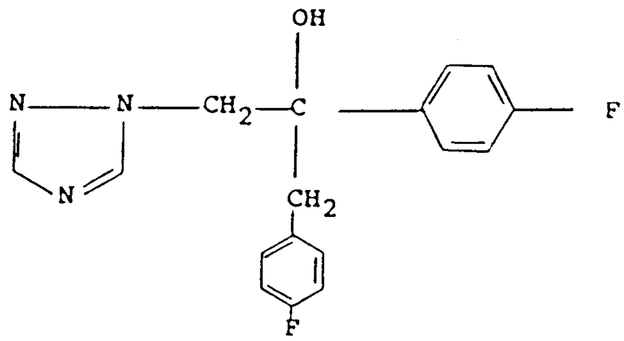

**38.** Procédé suivant la revendication 1, dans lequel le composé répond à la formule :

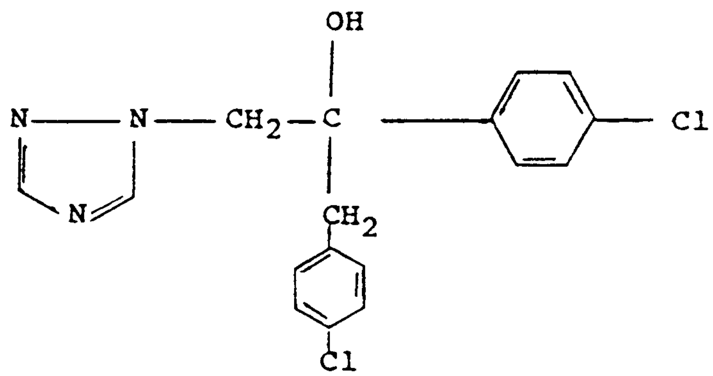

**39.** Procédé suivant la revendication 1, dans lequel le composé répond à la formule :

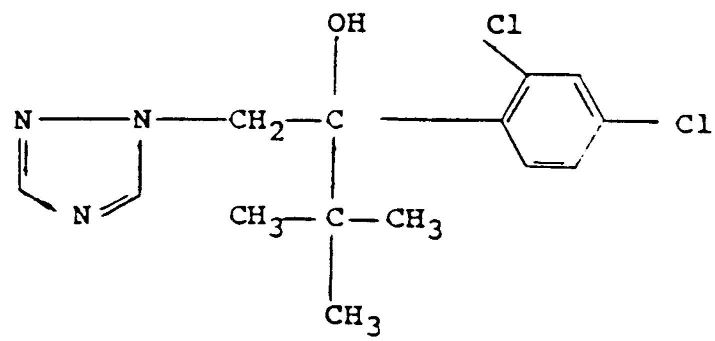

**40.** Procédé suivant la revendication 1, dans lequel le composé répond à la formule :

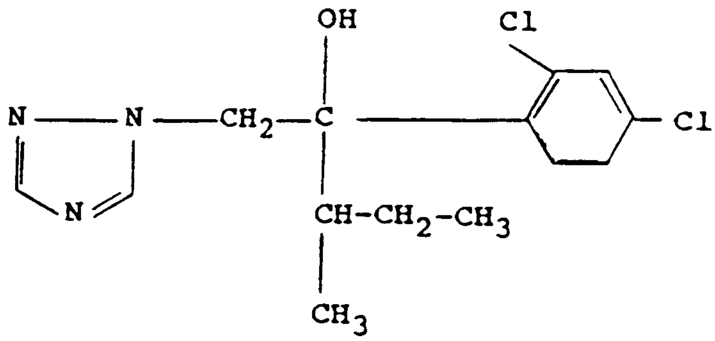

**41.** Procédé suivant la revendication 1, dans lequel le composé répond à la formule :

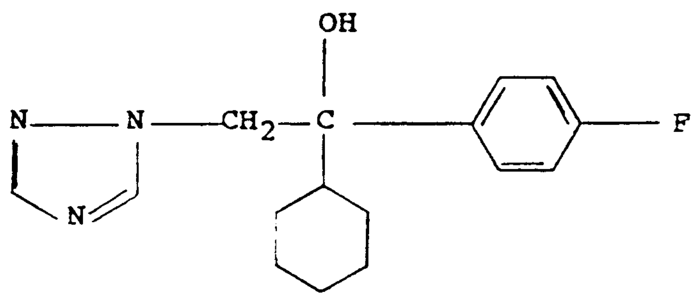

**42.** Procédé suivant la revendication 1, dans lequel le composé répond à la formule :

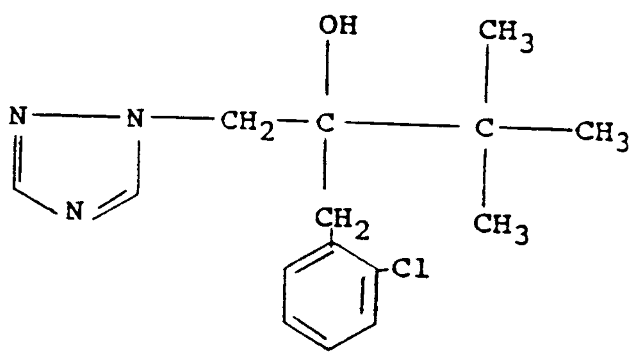

**43.** Procédé suivant la revendication 1, dans lequel le composé répond à la formule :

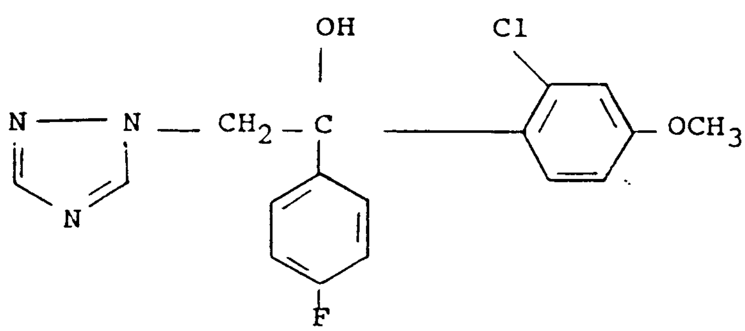

**44.** Bois, peaux, cuir et films de peinture traités par le procédé suivant la revendication 27.

**45.** Bois, peaux, cuir et films de peinture traités avec un composé tel que défini dans la revendication 1 ou un sel d'addition d'acide, un ester ou un complexe métallique de ce composé.

## Patentansprüche

**1.** Verfahren zum Bekämpfen von Pilzen auf nichtlandwirtschaftlichen Materialien, bei welchem auf das Material oder die Umgebung des Materials eine zur Erzielung eines pilzbekämpfenden Effekts ausreichende Menge einer Verbindung der allgemeinen Formel (I),

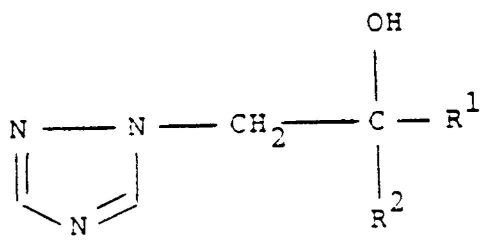

worin $R^1$ für $C_{1-6}$-Alkyl oder Cycloalkyl mit bis zu 6 Kohlenstoffatomen oder Phenyl steht und $R^2$ für Phenyl oder Benzyl steht; wobei das Phenyl von $R^1$ und das Phenyl oder der Phenyl-Teil des Benzyls von $R^2$ gegebenenfalls substituiert ist mit Halogen, $C_{1-5}$-Alkyl, $C_{1-4}$-Alkoxy, Halogeno-$C_{1-4}$-alkyl, Halogeno-$C_{1-4}$-alkoxy, Nitro, Phenyl, Phenoxy, Benzyl, gegebenenfalls halogenosubstituiertem Benzyloxy, Alkylendioxy, Halogenoalkylendioxy, Amino, Mono- oder Di-$C_{1-4}$-alkylamino, Hydroxy, Morpholino oder Carboxy oder einem Alkylester davon, und/oder der Alkyl-Teil des Benzyls gegebenenfalls substituiert ist mit einem $C_{1-2}$-Alkyl; oder ein Säureadditionssalz, ein Ester oder ein Metallkomplex davon, wie sie oben definiert sind, aufgebracht wird.

**2.** Verfahren nach Anspruch 1, bei welchem $R^1$ für $C_{1-4}$-Alkyl und $R^2$ für gegebenenfalls substituiertes Benzyl steht.

**3.** Verfahren nach Anspruch 1, bei welchem $R^1$ für gegebenenfalls substituiertes Phenyl und $R^2$ für gegebenenfalls substituiertes Benzyl steht.

**4.** Verfahren nach Anspruch 1, bei welchem $R^1$ und $R^2$, welche gleich oder verschieden sein können, für gegebenenfalls substituiertes Phenyl stehen.

**5.** Verfahren nach Anspruch 1, 2 oder 5, bei welchem $R^1$ für $C_{1-4}$-Alkyl und $R^2$ für gegebenenfalls substituiertes Phenyl steht.

**6.** Verfahren nach Anspruch 1, bei welchem $R^1$ für Butyl steht.

**7.** Verfahren nach Anspruch 6, bei welchem $R^1$ für t-Butyl steht.

**8.** Verfahren nach Anspruch 4, bei welchem $R^1$ und $R^2$ beide für Halogenophenyl stehen.

**9.** Verfahren nach Anspruch 8, bei welchem $R^1$ und $R^2$, welche gleich oder verschieden sein können, jeweils für Chlorophenyl oder Fluorophenyl stehen.

**10.** Verfahren nach Anspruch 8 oder 9, bei welchem mindestens ein Phenyl-Ring auch eine oder mehrere Alkoxy-Gruppen trägt.

**11.** Verfahren nach Anspruch 5, bei welchem $R^1$ für Alkyl und $R^2$ für Halogenophenyl steht.

**12.** Verfahren nach Anspruch 11, bei welchem $R^1$ für n-Butyl oder t-Butyl und $R^2$ für p-Chlorophenyl steht.

**13.** Verfahren nach Anspruch 10, bei welchem $R^1$ für 2-Chloro-4-methoxyphenyl und $R^2$ für 2-Chloro-, 2-Fluoro-, 4-Chloro- oder 4-Fluorophenyl steht.

**14.** Verfahren nach Anspruch 1, bei welchem die Verbindung die folgende Formel aufweist:

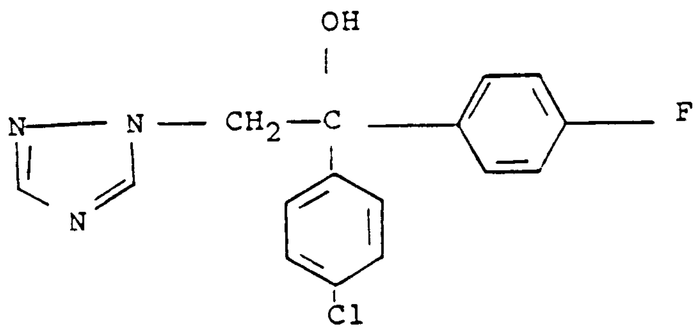

**15.** Verfahren nach Anspruch 1, bei welchem die Verbindung die folgende Formel aufweist:

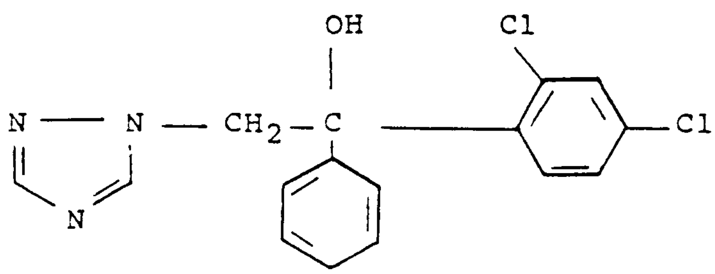

**16.** Verfahren nach Anspruch 1, bei welchem die Verbindung die folgende Formel aufweist:

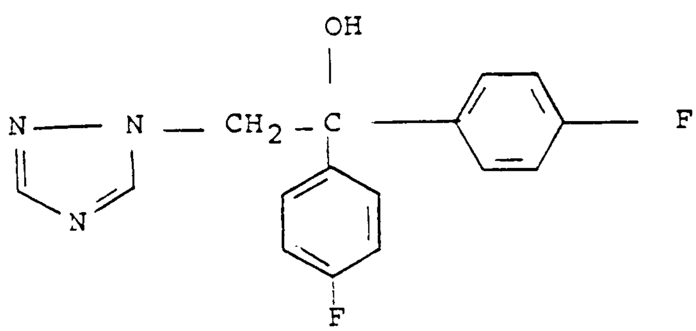

**17.** Verfahren nach Anspruch 1, bei welchem die Verbindung die folgende Formel aufweist:

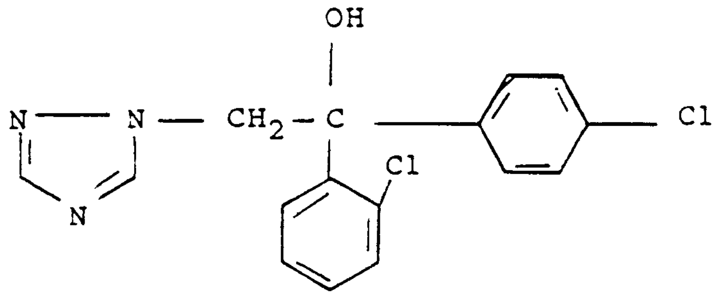

**18.** Verfahren nach Anspruch 1, bei welchem die Verbindung die folgende Formel aufweist:

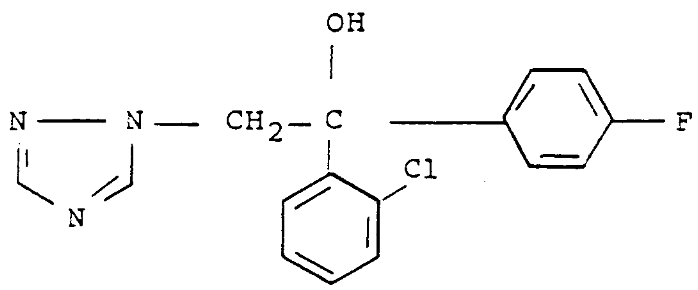

**19.** Verfahren nach Anspruch 1, bei welchem die Verbindung die folgende Formel aufweist:

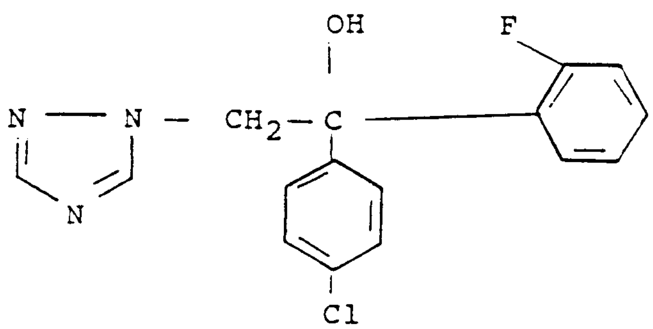

**20.** Verfahren nach Anspruch 1, bei welchem die Verbindung die folgende Formel aufweist:

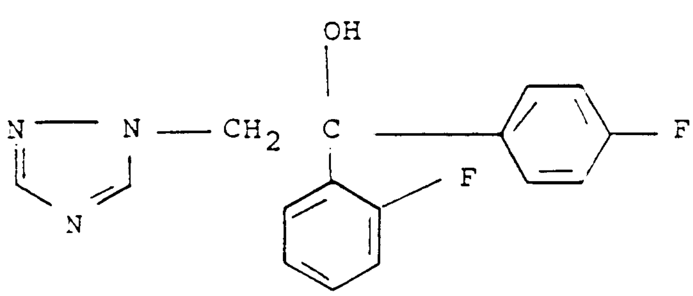

**21.** Verfahren nach Anspruch 1, bei welchem die Verbindung die folgende Formel aufweist:

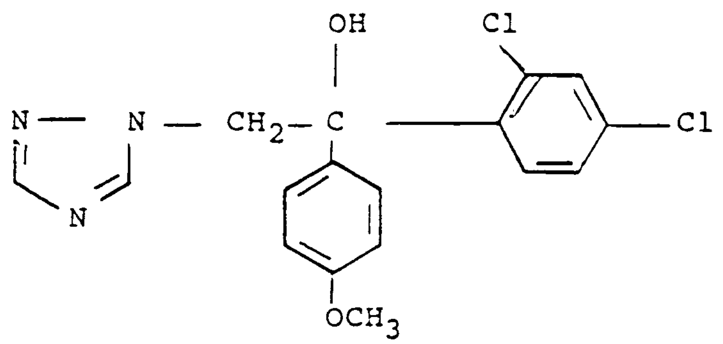

**22.** Verfahren nach Anspruch 1, bei welchem die Verbindung die folgende Formel aufweist:

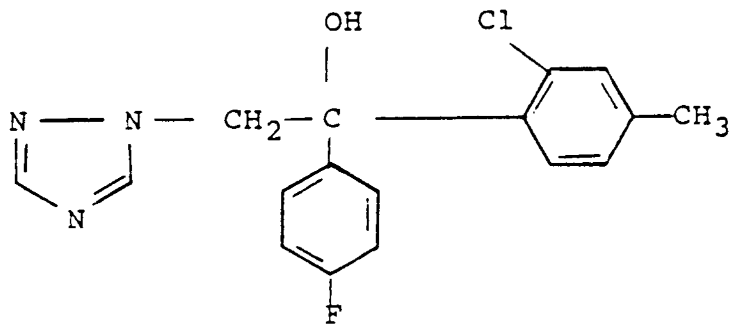

**23.** Verfahren nach Anspruch 1, bei welchem die Verbindung die folgende Formel aufweist:

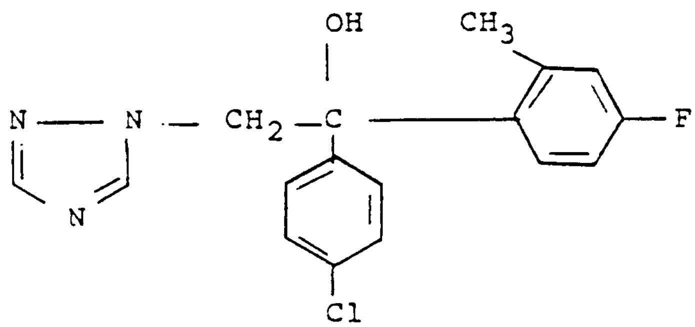

**24.** Verfahren nach Anspruch 1, bei welchem die Verbindung die folgende Formel aufweist:

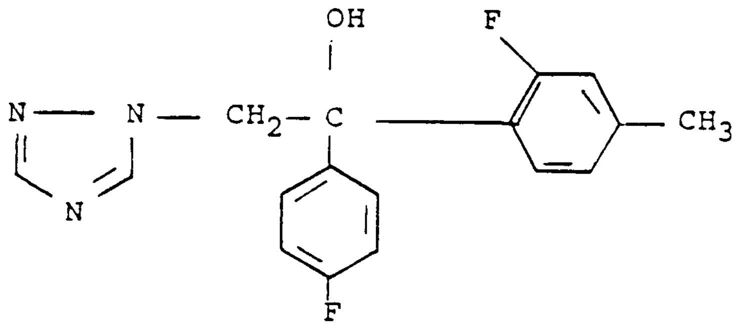

**25.** Verfahren nach Anspruch 1, bei welchem die Verbindung die folgende Formel aufweist:

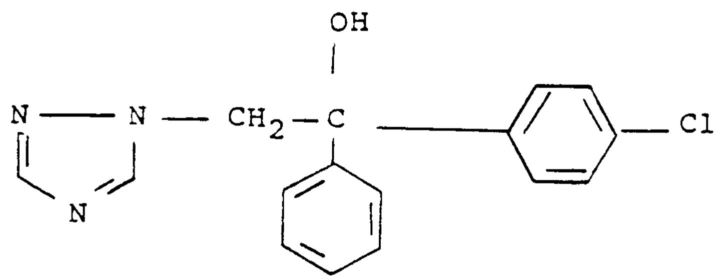

**26.** Verfahren nach Anspruch 1, bei welchem die Verbindung die folgende Formel aufweist:

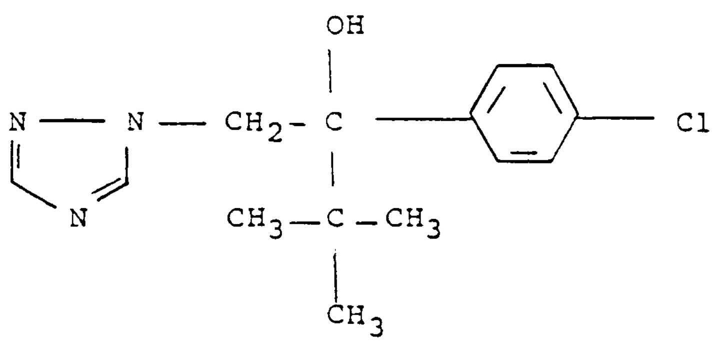

**27.** Verfahren nach Anspruch 1, bei welchem die Verbindung die folgende Formel aufweist:

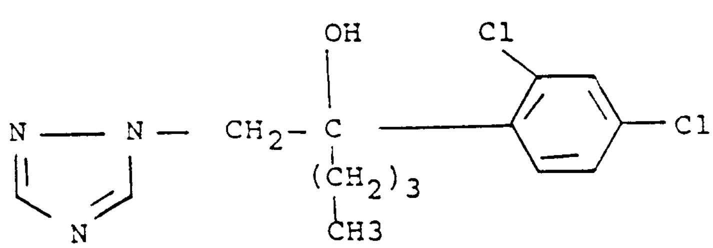

**28.** Verfahren nach Anspruch 1, bei welchem die Verbindung die folgende Formel aufweist:

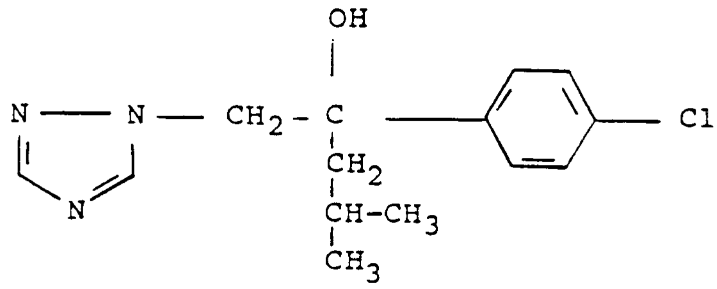

29. Verfahren nach Anspruch 1, bei welchem die Verbindung die folgende Formel aufweist:

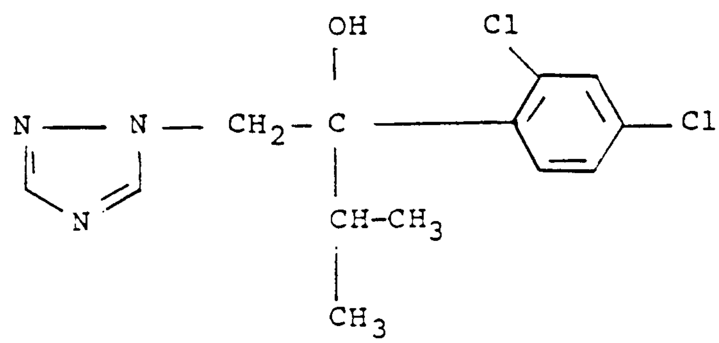

30. Verfahren nach Anspruch 1, bei welchem die Verbindung die folgende Formel aufweist:

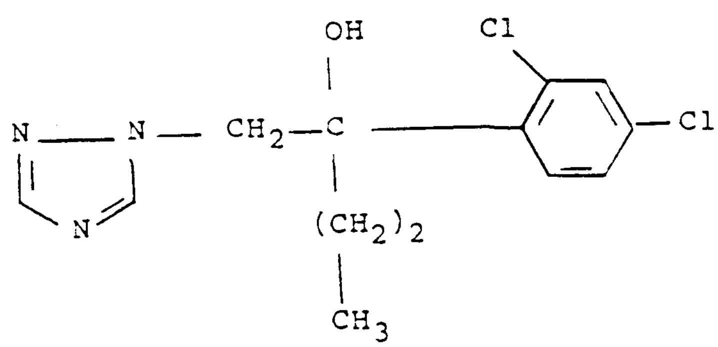

31. Verfahren nach Anspruch 1, bei welchem die Verbindung die folgende Formel aufweist:

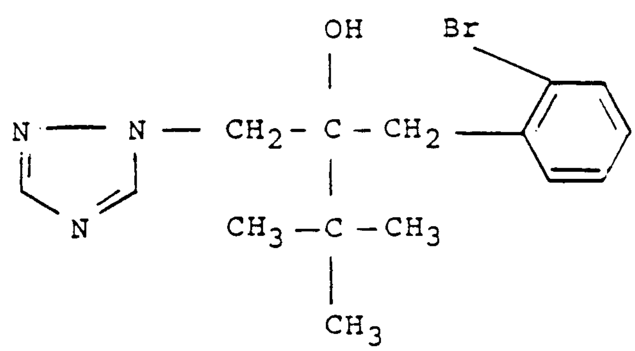

**32.** Verfahren nach Anspruch 1, bei welchem die Verbindung die folgende Formel aufweist:

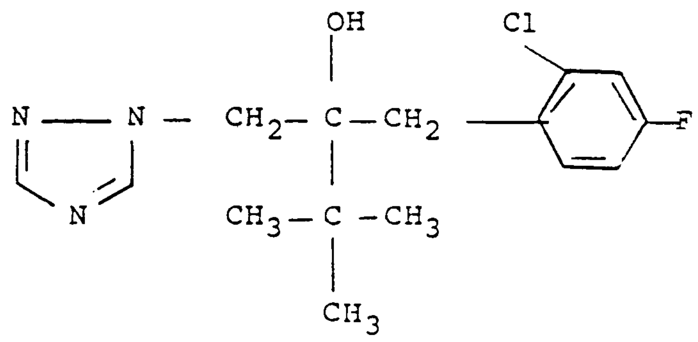

**33.** Verfahren nach Anspruch 1, bei welchem die Verbindung die folgende Formel aufweist:

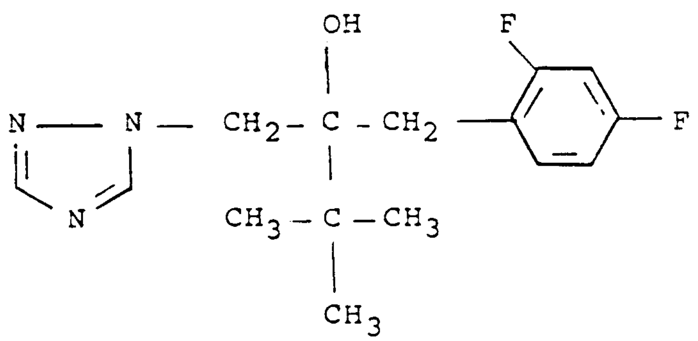

**34.** Verfahren nach Anspruch 1, bei welchem die Verbindung die folgende Formel aufweist:

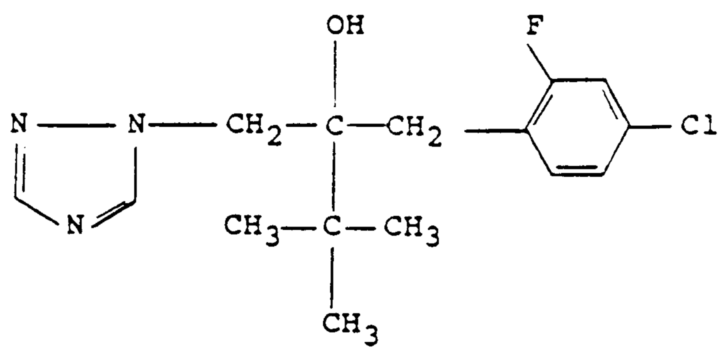

**35.** Verfahren nach Anspruch 1, bei welchem die Verbindung die folgende Formel aufweist:

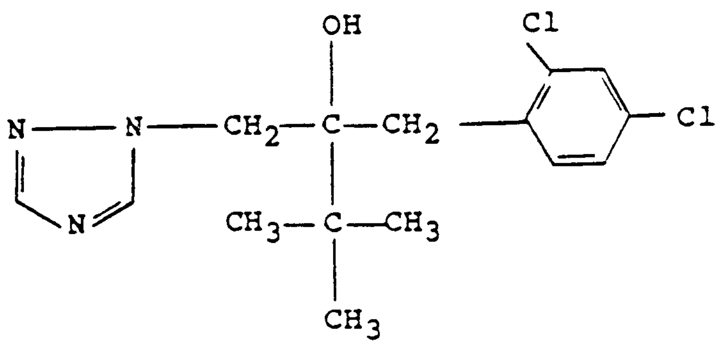

36. Verfahren nach Anspruch 1, bei welchem die Verbindung die folgende Formel aufweist:

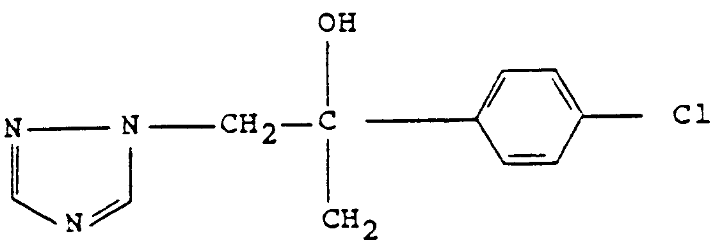

37. Verfahren nach Anspruch 1, bei welchem die Verbindung die folgende Formel aufweist:

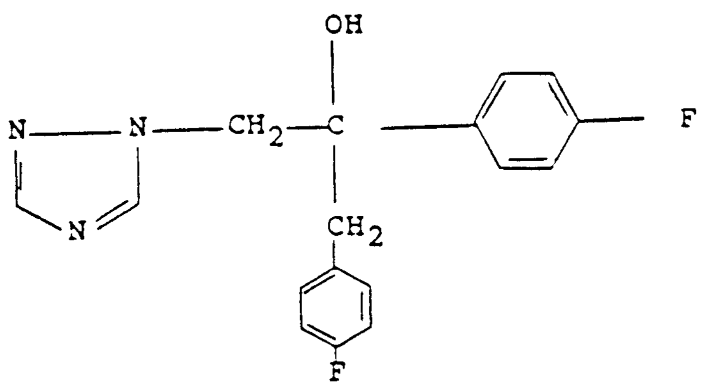

38. Verfahren nach Anspruch 1, bei welchem die Verbindung die folgende Formel aufweist:

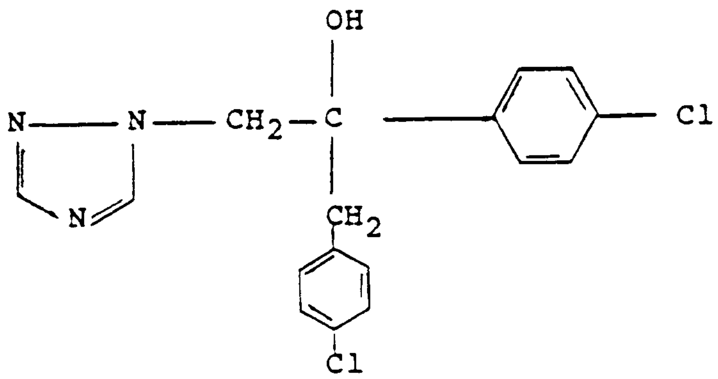

**39.** Verfahren nach Anspruch 1, bei welchem die Verbindung die folgende Formel aufweist:

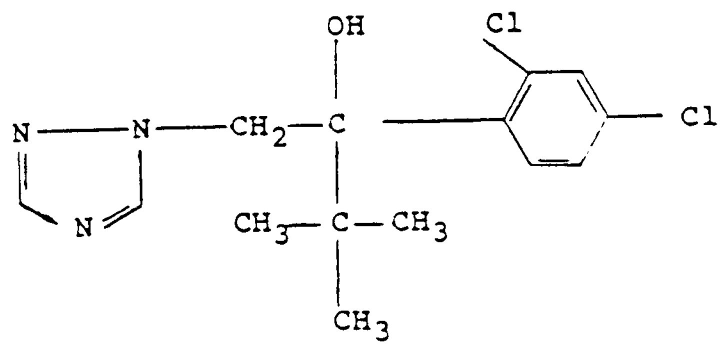

**40.** Verfahren nach Anspruch 1, bei welchem die Verbindung die folgende Formel aufweist:

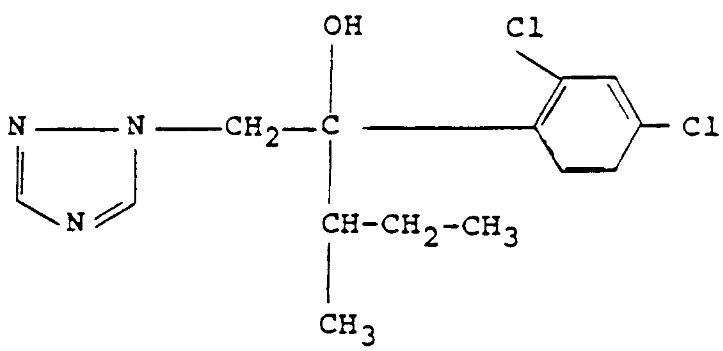

**41.** Verfahren nach Anspruch 1, bei welchem die Verbindung die folgende Formel aufweist:

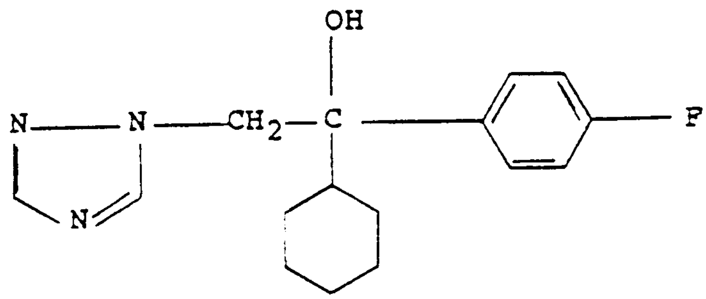

42. Verfahren nach Anspruch 1, bei welchem die Verbindung die folgende Formel aufweist:

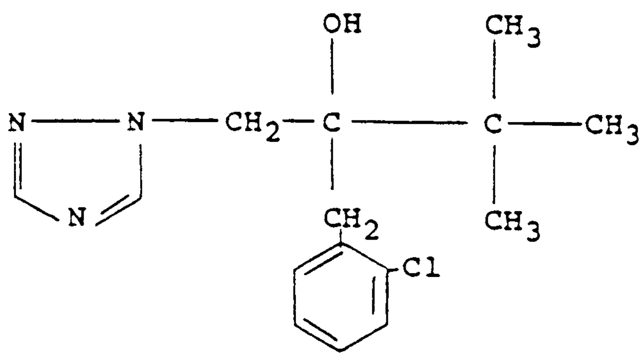

43. Verfahren nach Anspruch 1, bei welchem die Verbindung die folgende Formel aufweist:

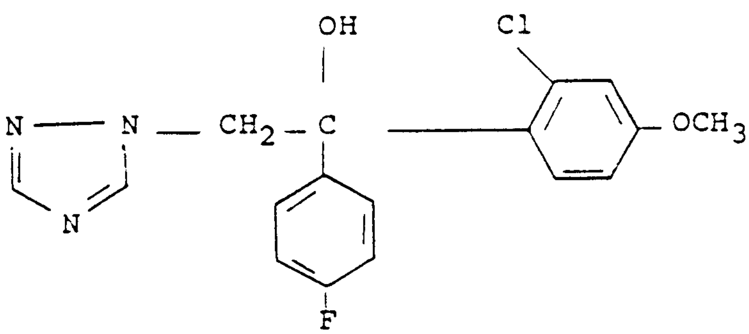

44. Holz, Häute, Leder und Anstrichfilme, sofern sie durch das Verfahren von Anspruch 27 behandelt worden sind.

45. Holz, Häute, Leder und Anstrichfilme, sofern sie mit einer Verbindung nach Anspruch 1 oder einem Säureadditionssalz, einem Ester oder einem Metallkomplex davon behandelt worden sind.